# EUROPEAN PATENT APPLICATION

(11) **EP 4 534 070 A1**
(43) Date of publication of application: **09.04.2025**
(21) Application number: 23812089.3
(22) Date of filing: 22.05.2023
(51) Int. Cl.: A61K 8/25, A61K 8/26, A61K 8/891, A61K 8/81, A61K 8/895, A61K 8/02, A61Q 1/02, B41M 7/00

(54) **TATTOO PRINTING METHOD, INK FIXER COMPOSITION FOR TATTOO PRINTING, INK COMPOSITION FOR TATTOO PRINTING, FIXER COMPOSITION FOR TATTOO PRINTING, AND TATTOO PRINTING KIT COMPRISING SAME**

(30) Priority: 26.05.2022 KR 20220065001; 21.02.2023 KR 20230023135; 24.02.2023 KR 20230025195; 03.04.2023 KR 20230043540
(71) Applicant: LG H&H Co., Ltd., Seoul 03184 (KR)
(72) Inventor: CHEONG, Hae Na, Seoul 07795 (KR); JEONG, Ji Hee, Seoul 07795 (KR); YOON, Han Sun, Seoul 07795 (KR); KIM, Young Hwan, Seoul 07795 (KR); KIM, Ji Hun, Seoul 07795 (KR)
(74) Representative: Goddar, Heinz J.
(86) International application number: PCT/KR2023/006910
(87) International publication number: WO 2023/229315

(57) **Abstract**

The present invention relates to a tattoo printing method in which an ink fixer composition for tattoo printing comprising powder can be applied before or after an ink composition for tattoo printing is applied. The present invention may comprise the steps of: applying a primer composition for tattoo printing or an ink fixer composition for tattoo printing onto the skin; and applying the ink composition for tattoo printing on the area where the primer composition for tattoo printing or the ink fixer composition for tattoo printing has been applied, and the present invention may comprise the steps of: applying an ink composition for tattoo printing onto the skin; and applying the fixer composition for tattoo printing on the area where the ink composition for tattoo printing has been applied.

## Description

### [Technical Field]

The present invention relates to a tattoo printing method, an ink fixer composition for tattoo printing, an ink composition for tattoo printing, a fixer composition for tattoo printing, and a tattoo printing kit including the same.

### [Background Art]

As part of self-expression, many people are showing interest in clothes, hair, accessories, and the like, and expressing their individuality by putting designs on their skin. Common methods of drawing images on the skin include face painting, makeup, water transfer stickers, instant stickers, tattoo stencils, stamps, natural dyeing, henna, and tattoos. Common tattoos, henna, or body painting have limited accessibility because they require the application of a professional, and stickers and tattoo stencils have the problem that only limited designs may be used. Therefore, there is a growing interest in skin printers that allow users to easily print desired images on the skin.

Meanwhile, the inkjet recording method is a method in which a document or image in digital data format is transmitted as an electric signal and printed on a medium by spraying fine ink droplets of various colors onto the paper through a head. Hundreds to thousands of fine ink spray ports (nozzles) are arranged on the head to spray fine ink droplets of 1 to 10 picoliters (pl) from each disposed position. The inkjet recording methods include electrostatic attraction methods to spray ink droplets using electrostatic attraction, pressure transfer methods using a piezoelectric element to impart mechanical vibration or displacement to an ink, and thermal transfer methods to generate bubbles by heating an ink and use the pressure at that time to eject the ink.

To print ink directly on the skin, harmless water-based inks may be mainly used, and water-based inks include 60% to 90% of water. When printed on a medium and colliding with it, a water-based ink dries while evaporation, infiltration, and diffusion occur simultaneously. In other words, the coloring matter of the ink remains on the medium, and the moisture infiltrates into the medium, or evaporates and dries. At this time, how the water-based ink dries and whether the coloring matter is fixed at the point where it falls have a major effect on the printing quality.

Generally, coloring matter in water-based inks do not remain on the surface of a medium, but infiltrate and settle in the inside to be printed. Therefore, when the infiltration of the moisture in the coloring matter into the printing medium is delayed, the ink may smudge on the surface of the medium and contamination of the ink color may occur. To achieve ideal printing quality, the colorant (dye or pigment) present in a water-based ink must remain in a circular form on the surface in large quantities to express vivid colors, and the moisture must dry quickly to prevent the water-based ink from moving in the plane direction.

On office paper or inkjet-only paper, vertical absorption of water-based inks occurs quickly, whereas on the skin, which is smooth and has an uneven surface and poor interaction with water-based inks, the infiltration of water-based inks does not occur smoothly on the skin, and more time is required for water-based inks to infiltrate on the skin than on common paper. In addition, the drying time of glycols and diols, which are the main solvents of water-based inks, is long on the skin, and they have poor spreading properties.

Therefore, there is a growing need for the development of inks, ink fixers (primers), or fixers (topcoats) that can be applied to the skin before or after printing to increase the skin absorption speed and drying speed of water-based inks, so that the inks can be printed without smudging and to achieve excellent print quality.

### [Disclosure]

### [Technical Problem]

The present invention has been invented to solve the above-described problems of the prior art, and an object the present invention is to provide a tattoo printing method, an ink fixer composition for tattoo printing, an ink composition for tattoo printing, a fixer composition for tattoo printing, and a tattoo printing kit including the same, which can realize excellent printing quality by preventing an ink from smudging on a skin when the ink is printed on the skin, for example, when an ink is directly printed on the skin with an inkjet printer, or when an ink is printed on a specific medium with an inkjet printer and the ink is printed on the skin using the medium.

The tasks of the present invention are not limited to the above-mentioned tasks, and other tasks that are not mentioned will be clearly understood by those skilled in the art from the description below.

### [Technical Solution]

In a tattoo printing method according to one aspect of the present invention, an ink fixer composition for tattoo printing including powder may be applied before an ink composition for tattoo printing is applied or after an ink composition for tattoo printing is applied.

Specifically, the ink fixer composition for tattoo printing may include a binder in an amount of 3% to 15% by weight based on the total weight of the ink fixer composition.

Specifically, the powder may include spherical powder or plate-shaped powder.

Specifically, the ink fixer composition for tattoo printing may include spherical powder in an amount of 54% to 89% by weight or plate-shaped powder in an amount of 5% to 35% by weight based on the total weight of the ink fixer composition.

Specifically, the plate-shaped powder may include one or more of talc, mica, synthetic fluorophlogopite, boron nitride, sericite, illite, perlite, montmorillonite, boron nitride, bismuth oxychloride, barium sulfate, and alumina.

Specifically, the spherical powder may have an average particle size of 3 to 10 µm.

Specifically, the spherical powder may include one or more of silica, polymethyl methacrylate, methyl methacrylate crosspolymer, nylon-12, polymethylsilsesquioxane, vinyl dimethicone/methicone silsesquioxane crosspolymer, diphenyl dimethicone/vinyl diphenyl dimethicone/silsesquioxane crosspolymer, and hexamethylene diisocyanate (HDI)/trimethylol hexyllactone crosspolymer.

Specifically, the spherical powder may include an absorbent spherical powder.

Specifically, the absorbent spherical powder may include silica.

Specifically, the absorbent spherical powder may include 16% or more of spherical powder having a sum of a water absorption amount and an oil absorption amount of 4 ml/g or more based on the total weight of the spherical powder.

Specifically, the absorbent spherical powder may have a sum of a water absorption amount and an oil absorption amount of 0.8 ml/g or more.

Specifically, the absorbent spherical powder may have a water absorption amount of 0.4 ml/g or more and an oil absorption amount of 0.45 ml/g or more.

An ink fixer composition for tattoo printing according to one aspect of the present invention may include powder.

Specifically, the ink fixer composition for tattoo printing may include a binder in an amount of 3% to 15% by weight.

Specifically, the powder may include spherical powder or plate-shaped powder.

Specifically, the ink fixer composition for tattoo printing may include spherical powder in an amount of 54% to 89% by weight or plate-shaped powder in an amount of 5% to 35% by weight based on the total weight of the ink fixer composition.

Specifically, the plate-shaped powder may include one or more of talc, mica, synthetic fluorophlogopite, boron nitride, sericite, illite, perlite, montmorillonite, boron nitride, bismuth oxychloride, barium sulfate, and alumina.

Specifically, the spherical powder may have an average particle size of 3 to 10 µm.

Specifically, the spherical powder may include one or more of silica, polymethyl methacrylate, methyl methacrylate crosspolymer, nylon-12, polymethylsilsesquioxane, vinyl dimethicone/methicone silsesquioxane crosspolymer, diphenyl dimethicone/vinyl diphenyl dimethicone/silsesquioxane crosspolymer, and HDI/trimethylol hexyllactone crosspolymer.

Specifically, the spherical powder may include an absorbent spherical powder.

Specifically, the absorbent spherical powder may include silica.

Specifically, the absorbent spherical powder may include 16% or more of spherical powder having a sum of a water absorption amount and an oil absorption amount of 4 ml/g or more based on the total weight of the spherical powder.

Specifically, the absorbent spherical powder may have a sum of a water absorption amount and an oil absorption amount of 0.8 ml/g or more.

Specifically, the absorbent spherical powder may have a water absorption amount of 0.4 ml/g or more and an oil absorption amount of 0.45 ml/g or more.

A tattoo printing kit according to one aspect of the present invention may include: the ink fixer composition for tattoo printing; and a tattoo printing device including an ink composition for tattoo printing.

Specifically, the tattoo printing kit may further include a top coat composition for tattoo printing or a fixer composition for tattoo printing.

A tattoo printing method according to one aspect of the present invention may include: applying the ink fixer composition for tattoo printing to a skin; and applying an ink composition for tattoo printing on top of an area on which the ink fixer composition for tattoo printing has been applied.

Specifically, the tattoo printing method may further include applying a top coat composition for tattoo printing or a fixer composition for tattoo printing on top of an area on which the ink composition for tattoo printing has been applied.

An ink composition for tattoo printing according to one aspect of the present invention may include: one or more humectant selected from glycerin and polyethylene glycol (PEG)-8; one or more surfactant selected from polyglyceryl-4 caprate and PEG-7 glyceryl cocoate; or one or more antifoamer selected from glycereth-26 and a plant-derived oil.

Specifically, the ink composition for tattoo printing may include the humectant in an amount of 10% to 40% by weight based on the total weight of the ink composition.

Specifically, the ink composition for tattoo printing may include the surfactant in an amount of 0.001% to 10% by weight based on the total weight of the ink composition.

Specifically, the ink composition for tattoo printing may include the antifoamer in an amount of 0.001% to 10% by weight based on the total weight of the ink composition.

Specifically, the plant-derived oil may include one or more of hydrogenated ethylhexyl olivate and hydrogenated olive oil unsaponifiable.

Specifically, the plant-derived oil may be included in an amount of 0.001% to 10% by weight based on the total weight of the ink composition.

Specifically, the ink composition may be a water-based ink.

Specifically, the ink composition may further include a colorant including a dye or a pigment.

Specifically, the colorant may be included in an amount of 0.1% to 10% by weight based on the total weight of the ink composition.

A tattoo printing method according to one aspect of the present invention may include: applying a primer composition for tattoo printing or an ink fixer composition for tattoo printing on a skin; and applying the ink composition for tattoo printing on top of an area on which the primer composition for tattoo printing or the ink fixer composition for tattoo printing has been applied.

Specifically, the tattoo printing method may further include: applying a top coat composition for tattoo printing or a fixer composition for tattoo printing on top of an area on which the ink composition for tattoo printing has been applied.

Specifically, a tattoo printing method according to one aspect of the present invention may include: applying the ink composition for tattoo printing on a skin; and applying a top coat composition for tattoo printing or a fixer composition for tattoo printing on top of an area on which the ink composition for tattoo printing has been applied.

A tattoo printing device according to one aspect of the present invention may include the ink composition for tattoo printing.

A tattoo printing kit according to one aspect of the present invention may include the tattoo printing device; and a primer composition for tattoo printing or an ink fixer composition for tattoo printing.

Specifically, the tattoo printing kit may further include a top coat composition for tattoo printing or a fixer composition for tattoo printing.

A tattoo printing kit according to one aspect of the present invention may include the tattoo printing device; and a top coat composition for tattoo printing or a fixer composition for tattoo printing.

In a tattoo printing method according to one aspect of the present invention, a fixer composition for tattoo printing in any one formulation of a balm type, a jelly type, a solid type, and a semi-solid type may be applied before an ink composition for tattoo printing is applied or after an ink composition for tattoo printing is applied.

Specifically, the fixer composition for tattoo printing may include a hydrocarbon-based oil or wax.

Specifically, the hydrocarbon oil or wax may include one or more of petrolatum, synthetic wax, ceresin, and microcrystalline wax.

Specifically, the fixer composition for tattoo printing may include the hydrocarbon-based oil or wax in an amount of 65% to 100% by weight based on the total weight of the fixer composition.

A fixer composition for tattoo printing according to one aspect of the present invention may be in any one formulation of a balm type, a jelly type, a solid type, and a semi-solid type.

Specifically, the fixer composition for tattoo printing may include a hydrocarbon-based oil or wax.

Specifically, the hydrocarbon oil or wax may include one or more of petrolatum, synthetic wax, ceresin, and microcrystalline wax.

The fixer composition for tattoo printing may include the hydrocarbon-based oil or wax in an amount of 65% to 100% by weight based on the total weight of the fixer composition.

A tattoo printing kit according to one aspect of the present invention may include: the fixer composition for tattoo printing; and a tattoo printing device including an ink composition for tattoo printing.

Specifically, the tattoo printing kit may further include a primer composition for tattoo printing or an ink fixer composition for tattoo printing.

A tattoo printing method according to one aspect of the present invention may include: applying an ink composition for tattoo printing to a skin; and applying the fixer composition for tattoo printing on top of an area on which the ink composition for tattoo printing has been applied.

Specifically, the ink composition for tattoo printing may be applied on top of an area on which a primer composition for tattoo printing or an ink fixer composition for tattoo printing has been applied.

Preferably, an ink fixer composition according to the present invention is preferably used before an ink for tattoo printing is printed on a skin, and a conventional fixing agent known in the art may be used after the ink is printed on the skin. Of course, an ink fixer composition according to the present invention may be used after an ink is printed on a skin.

The present invention also provides a fixer composition used after an ink is printed on a skin. A fixer composition used after an ink according to the present invention printed on a skin may be a transparent formulation having a viscosity such fixer composition does not substantially flow off without an external force, and may preferably be a transparent balm type, a jelly type, a solid type, or a semi-solid type having a hardness that allows the fixer composition to be spread at room temperature, and the fixer composition may be made of, for example, petrolatum or contain a large amount of petrolatum.

Preferably, a fixer composition according to the present invention may preferably be used after a tattoo printing ink is printed on a skin, and a conventional fixing agent known in the art may be used before an ink is printed on a skin. Of course, a fixer composition according to the present invention may be used before an ink is printed on a skin.

In addition, the present invention provides a tattoo printing kit including an ink fixer composition including the above-described powder, an ink for tattoo printing, and a fixer composition used after the ink is printed on a skin.

The present invention provides a use of at least one of an ink fixer composition for tattoo printing, an ink composition for tattoo printing, a fixer composition for tattoo printing, and a tattoo printing kit including the same according to one aspect of the present invention. Specifically, the present invention provides a use of at least one of an ink fixer composition for tattoo printing, an ink composition for tattoo printing, a fixer composition for tattoo printing, and a tattoo printing kit including the same according to one aspect of the present invention in skin printing or tattoo printing.

Specifically, the use may include printing an image or the like directly on a skin of a subject with an ink composition, or printing an image or the like on a medium such as paper with an ink composition and printing the image or the like on the skin of a subject using the medium.

Specifically, an ink composition for tattoo printing may be used for printing an image or the like on a skin or on a medium. In addition, before an image or the like is printed on a skin or medium with an ink composition, a primer composition or ink fixer composition for tattoo printing may be applied to the skin or medium to have the use of improving the printing performance of an ink composition, such as increasing the fixing ability of the ink composition. In addition, after an image or the like is printed on a skin or medium with an ink composition, a topcoat composition or a fixer composition for tattoo printing may be applied to the skin or medium to have the use of improving the printing performance of an ink composition, such as increasing the fixing ability of the ink composition.

### [Advantageous Effects]

A tattoo printing method, an ink fixer composition for tattoo printing, an ink composition for tattoo printing, a fixer composition for tattoo printing, and a tattoo printing kit according to the present invention can improve the printing performance of the ink composition and the skin fixing ability of the ink composition.

The effects of the present invention are not limited to the above-mentioned effects, and other effects that are not mentioned can be clearly understood by those skilled in the art from the description of the claims.

### [Description of Drawings]

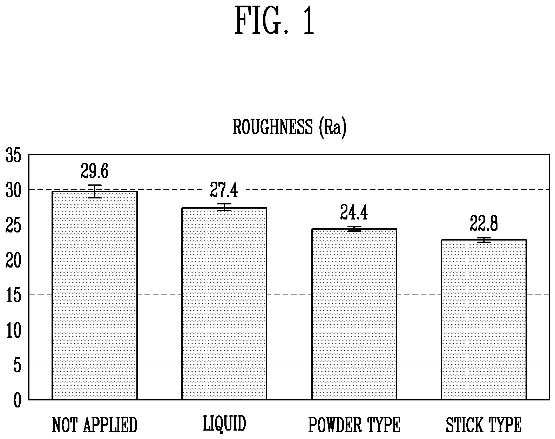

FIG. 1 shows a graph illustrating the roughness values of an artificial skin surface according to the type of ink fixer composition for tattoo printing.

### [Modes of the Invention]

Hereinafter, when a part is described to "include" a certain component, this does not mean excluding other components, but rather may include other components, unless otherwise specified.

Hereinafter, when the amount of a certain component is expressed as %, this may represent % by weight, unless otherwise specified.

Hereinafter, when it is described that "one or more" or "at least one" is included, this means not only including one component, but also including one component and other components together, unless otherwise specified.

Hereinafter, the present invention will be described in more detail for the purpose of understanding the present invention.

Meanwhile, each description and embodiment disclosed herein may also be applied to each other description and embodiment. In other words, all combinations of various elements disclosed herein fall within the scope of the present invention. In addition, the scope of the present invention is not limited by the specific description described below.

In addition, a person skilled in the art will recognize or confirm many equivalents to the specific embodiments of the present invention described herein, using only routine experimentation. In addition, such equivalents are intended to be included in the present invention.

A skin printing device (tattoo printing device) herein includes, for example, a skin printer (tattoo printer) for printing an ink composition on a skin, and the skin printing device is for printing a specific image on a skin using an ink composed of cosmetic raw materials applicable to a skin, and it may print a clear image on a skin by maintaining a constant distance between the skin and a nozzle, but the present invention is not limited thereto. In addition, a skin printing device herein is for printing an ink on a medium such as paper or film or on skin, and the present invention is not limited to the type of the device.

The terms "tattoo printing, tattoo printer, tattoo printing device" and "skin printing, skin printer, skin printing device" herein may be used to mean printing an ink a on skin, and may be used in the meaning of including a device for printing an ink on a medium such as paper, or a device for printing ink on a medium such as paper and using the medium to print on a skin. The terms "skin printing, skin printer, skin printing device" have the same meaning as the terms "tattoo printing, tattoo printer, tattoo printing device," respectively, and may be used herein interchangeably.

The terms "ink, fixer, ink fixer," and the like used herein may have the same meaning as "ink composition, fixer composition, ink fixer composition," respectively, and may be used herein interchangeably. In addition, "ink composition, fixer composition, ink fixer composition" may have the same meaning as "ink composition for tattoo printing, fixer composition for tattoo printing, ink fixer composition for tattoo printing," and may be used herein interchangeably.

A fixer composition herein is a material that is applied to a skin before or after printing to prevent an ink from coming into contact with the outside or being contaminated by an external substance, and the fixer composition can fix an ink to a skin without smudging and realize excellent printing quality. Preferably, a fixer composition may be used after applying an ink composition, but is not limited thereto.

A topcoat composition herein is a material used to protect a surface on which an ink composition is printed after the ink composition is printed, and may be used for the same purpose as a fixer composition and may include same components. A topcoat composition may be used without limitation as long as it is a material commonly used to coat an ink composition, and the present invention is not limited thereto.

An ink fixer herein is a material that is applied to a skin before or after printing an ink to increase the skin absorption rate and drying rate of the ink and to promote the formation of a coating layer, and an ink fixer may enable an ink to be printed on a skin without smudging and realize excellent printing quality. Preferably, an ink fixer composition may be used before application of an ink composition, but is not limited thereto.

A primer composition herein is a material that is applied on a skin or medium to form a layer in which an ink is accommodated by the skin or medium, and the ink may be accommodated by the primer layer formed on the skin or medium, and the fixing ability of an ink can be improved compared to a skin or medium to which a primer composition is not applied, and the durability of an printed image can be improved. In particular, a primer composition can improve the interaction with an aqueous ink so that a medium that is not suitable for printing an aqueous ink may accommodate the aqueous ink. A primer composition may be used for a same purpose as a fixer composition and may include same components. A primer composition may be used without limitation as long as it is a material commonly used to improve the ink accommodation of a surface on which an ink composition is applied, and the present invention is not limited thereto.

An ink fixer composition for tattoo printing of the present invention is intended to be applied to a skin before or after spraying an ink, and it can reduce a foreign feeling (white turbidity, etc.) when applied to the skin and compactly fill in fine wrinkles by relatively reducing the content of plate-shaped powder and increasing the content of spherical powder. Powder may be used without particular limitation as long as it is used in a conventional cosmetic, and the powder may include spherical powder or plate-shaped powder.

Spherical powder exhibits a smudging alleviation effect when the amount thereof is 50% by weight or more based on the total amount of an ink fixer composition, and in particular, the effect is particularly excellent when the amount of spherical powder is 54% to 89% by weight based on the total weight of an ink fixer composition. Plate-shaped powder may be further included to improve the skin adhesion of spherical powder and facilitate skin fixation of a print.

Plate-shaped powder may be selected from the group consisting of talc, mica, synthetic fluorophlogopite, sericite, illite, perlite, montmorillonite, boron nitride, bismuth oxychloride, barium sulfate, and alumina.

An ink fixer composition for tattoo printing of the present invention preferably includes 54% to 89% by weight of spherical powder based on the total weight of an ink fixer composition, or 5% to 35% by weight of plate-shaped powder based on the total weight of an ink fixer composition. Spherical powder may be more preferably included in an amount of 59% to 84% by weight of spherical powder based on the total weight of an ink fixer composition. Plate-shaped powder may be more preferably included in an amount of 10% to 30% by weight based on the total weight of an ink fixer composition.

In addition, a binder may be additionally blended to prevent powdering or further improve skin adhesion of powder, or the like. The binder is not particularly limited as long as it is commonly used in cosmetics, and diols, ester oils, hydrocarbon oils, silicone oils, and the like may be used. A binder is preferably included in an amount of 3% to 15% by weight based on 100% by weight of an ink fixer composition for tattoo printing. More preferably, a binder may be included in an amount of 5% to 10% by weight.

Spherical powder of an ink fixer composition for tattoo printing of the present invention has an excellent smudging alleviation effect when the average particle size is small. The average particle size of spherical powder is 12 µm or less, preferably 3 to 10 µm, and more preferably 5 to 9 µm.

Spherical powder is preferably composed of a component having excellent water absorption or oil absorption. A component having excellent water absorption or oil absorption is effective in preventing the smudging of an aqueous ink, and in particular, an absorbent component having excellent water absorption and oil absorption has the greatest effect in preventing the smudging of an aqueous ink.

Spherical powder is preferably selected from the group consisting of silica, polymethyl methacrylate, methyl methacrylate crosspolymer, nylon-12, polymethylsilsesquioxane, vinyl dimethicone/methicone silsesquioxane crosspolymer, diphenyl dimethicone/vinyl diphenyl dimethicone/silsesquioxane crosspolymer, and HDI/trimethylol hexyllactone crosspolymer. The absorbent component is preferably silica.

Silica may form a coating layer having a hydrophilic surface and porosity to improve the absorption of an ink, so it is preferable to use silica as a component of spherical powder. In particular, it is preferable to use absorbent silica having excellent hydrophilicity and lipophilicity, and a sum of a water absorption amount and an oil absorption amount may be 0.8 ml/g or more, and an absorbent silica preferably has a water absorption amount of 0.4 ml/g or more and an oil absorption amount of 0.45 ml/g or more, and an absorbent silica preferably has an absorption amount of 0.4 ml/g to 2.8 ml/g and an oil absorption amount of 0.45 ml/g to 4 ml/g. An ink fixer composition for tattoo printing of the present invention may preferably include 16% or more, 26% or more, 36% or more, or 46% or more of spherical powder having a sum of a water absorption amount and an oil absorption amount of 4 ml/g or more based on the total amount of the spherical powder.

An ink herein may include a colorant used for printing, painting, writing, and the like, and the colorant is used as a general term for a liquid colorant or a solid colorant. A colorant may include a pigment or a dye, and a colorant may preferably include a water-based dye.

A water-based ink is a composition including a water-soluble coloring matter as a main component, and any component commonly used in cosmetics may be used without limitation. When an ink is used in a printing device, the viscosity of the ink is preferably 2 to 5 cps (25 degrees, Enhanced UL Adapter, 60 rpm, Brookfield LTV) to prevent nozzle clogging, improve ink discharge, and improve the usability of a printer.

An ink composition according to one embodiment of the present invention is preferably a water-based ink using water as a solvent, and an ink composition may include components commonly used in an ink, such as a colorant, a humectant, a viscosity modifier, a surface tension modifier, an antifoamer, a preservative, and a surfactant, without limitation. In particular, an ink composition according to one embodiment of the present invention preferably includes a humectant, a surfactant, or an antifoamer.

An ink composition according to one embodiment of the present invention may include a colorant, and the colorant may preferably be included in an amount of 0.1% to 10% by weight based on the total weight of the ink composition. At this time, a dye or pigment may be used as the colorant, and the dye may preferably be an acid dye that may be used in cosmetics in the red, blue, and yellow series. For example, the dyes include Yellow No. 203, Yellow No. 4, Yellow No. 5, Yellow No. 201, Yellow No. 204, Red No. 103, Red No. 201, Red No. 202, Red No. 218, Red No. 220, Red No. 223, Red No. 226, Red No. 227, Blue No. 1, Blue No. 2, and the like. However, the present invention is not limited by the colorant included in the ink composition.

An ink composition according to one embodiment of the present invention may include a humectant. A humectant included in an ink composition may improve the moisturizing properties of an ink composition in a nozzle of a tattoo printer (skin printer), thereby preventing the nozzle from being clogged by an ink crust that is generated when the ink composition is sprayed and dried (nozzle clogging), and may improve the decap performance (the ability of an ink composition to be easily sprayed from a print head when exposed to the air). As a humectant, any humectant that may be used as a humectant in cosmetics may be used without limitation, but preferably, any one or more of glycerin and PEG-8 may be used. Preferably, a humectant may be included in an amount of 10% to 40% by weight, more preferably 15% to 30% by weight based on the total weight of the ink composition.

An ink composition according to one embodiment of the present invention may include a surfactant, and the surfactant may enable the ink composition to be quickly absorbed into a sponge provided for storing the ink composition, and may suppress the formation of bubbles in the ink composition during a printing process. As a surfactant, any component that may be used as a surfactant in cosmetics may be used without limitation, but preferably, any one or more of polyglyceryl-4 caprate and PEG-7 glyceryl cocoate may be used. A surfactant may be included in an amount of 0.001% to 10% by weight based on the total weight of an ink composition. Preferably, a surfactant may be included in an amount of 0.01% to 5% by weight.

An ink composition according to one embodiment of the present invention may include an antifoamer. An antifoamer can destroy bubbles included in an ink composition to improve the discharge stability of the ink composition. As an antifoamer, any component that may function as an antifoamer in cosmetics may be used without limitation, but preferably, any one or more of glycereth-26 and a plant-derived oil may be used. An antifoamer may be included in an amount of 0.001% to 10% by weight based on the total weight of an ink composition. Preferably, an antifoamer may be included in an amount of 0.01% to 5% by weight.

As a plant-derived oil, any one or more of hydrogenated ethylhexyl olivate and hydrogenated olive oil unsaponifiable may be used, and the plant-derived oil may be included in an amount of 0.001% to 10% by weight based on the total weight of an ink composition. Preferably, a plant-derived oil may be included in an amount of 0.1% to 5% by weight.

According to one embodiment of the present invention, a fixer composition may be in any one formulation of a balm type, a jelly type, a solid type, and a semi-solid type. A fixer composition may fix an ink composition on a skin. As a fixer composition, any component that may serve as a fixer composition in cosmetics may be used without limitation, but preferably, a hydrocarbon oil or wax may be used as a fixer composition. A hydrocarbon oil or wax may include any one or more of petrolatum, synthetic wax, ceresin, and microcrystalline wax, and a fixer composition may include 65% to 100% of the hydrocarbon oil or wax based on the total weight of the fixer composition. Preferably, a hydrocarbon oil or wax may be included in an amount of 65% to 95% by weight.

To further explain embodiments of the present disclosure, the following experimental examples are presented. These experimental examples are provided for illustrative purposes and should not be construed as limiting the scope of the disclosed embodiments.

### Experimental Example 1: Confirmation of degree of white turbidity occurrence and smudging alleviating effect according to the types of raw materials of ink fixer composition for tattoo printing

Since a powder cosmetic composition is used for makeup (makeup effect, blemish covering), powder with a large white turbidity effect (phenomenon in which the skin turns white) is mainly used as the powder cosmetic composition. However, when white turbidity occurs when an ink fixer composition for tattoo printing is used, the printed image may be blurred due to the white turbidity, which may deteriorate the printing quality. Therefore, it is preferable that an ink fixer composition does not cause white turbidity.

Each of A-I powder raw materials was applied to the forearm area using the Rubycell puff, and white turbidity was evaluated visually (H = high, M = medium, L = low).

The degree of white turbidity was relatively compared by measuring the haze and straight transmittance (haze: the higher the haze, the more white turbidity and the more opaque; straight transmittance (parallel transmittance): the higher straight transmittance, the less white turbidity and the more transparent).

Haze is a phenomenon in which light is diffused when passing through a transparent material according to the inherent properties of the material, in addition to reflection or absorption, resulting in an opaque, cloudy appearance. Haze is defined as the percentage of diffusion of a light incident on a transparent material.

Straight transmittance may be the percentage of light transmitted in the same direction as the incident direction for light incident on a transparent material. For example, the percentage of light that has penetrated a transparent material in a direction parallel to the normal direction, from the light incident in the direction parallel to the normal direction of the transparent material may be defined as the transmittance.

Each raw material was applied to a skin, and printing was performed using a skin printing device on an area where the raw material was applied. The ink used for printing was an ink for tattoo printing that included a humectant in an amount of 25% by weight, included one or more surfactants selected from polyglyceryl-4 caprate and PEG-7 glyceryl cocoate, and further included one or more antifoamers selected from glycereth-26, hydrogenated ethylhexyl olivate, and hydrogenated olive oil unsaponifiable. At this time, the smudging that occurred at a printing boundary was observed to evaluate the smudging alleviation effect of an ink fixer composition for tattoo printing.

As shown in Table 1 below, the degree of white turbidity occurrence and smudging alleviating effect were confirmed according to the types of raw materials.

Smudging alleviation evaluation method: ⊚: No ink smudging when observed visually; ∘: Ink smudged into fine wrinkles with a clear boundary when observed visually; △: Ambiguous boundary due to ink smudging when observed visually; and -: No effect.

**[Table 1]**

| **Raw material** | **Component** | **Manufacturer** | **Type** | **White turbidit y** | **Haz e (%)** | **Straight transmittan ce (%)** | **Smudgin g alleviatin g effect** |
|---|---|---|---|---|---|---|---|
| A | Synthetic fluorophlogopite | TOPY INDUSTRIES, LIMITED | Plate-like | H | 57.2 8 | 30.13 | Δ |
| B | Alumina | K.S. PEARL | | M | 47.7 2 | 39.83 | Δ |
| C | Talc, dimethicone, triethoxycapryly lsilane | K.S. PEARL | | M | 48.3 2 | 40.25 | Δ |
| D | Talc | ASADA MILLING | | H | 61.3 7 | 28.05 | Δ |
| E | Talc | NIPPON TALC | | H | 50.1 8 | 40.61 | Δ |
| F | Silica, methicone | ASAHI GLASS COMPANY | Spherical | L | 16.9 9 | 72.28 | ○ |
| G | Silica | ASAHI GLASS COMPANY | | L | 16 | 73.19 | ⊚ |
| H | Silica | SUNJIN CHEMICAL | | L | 25.4 6 | 63.1 | ○ |
| I | Silica | ABC NANOTECH | | L | 20.7 3 | 68.15 | ○ |

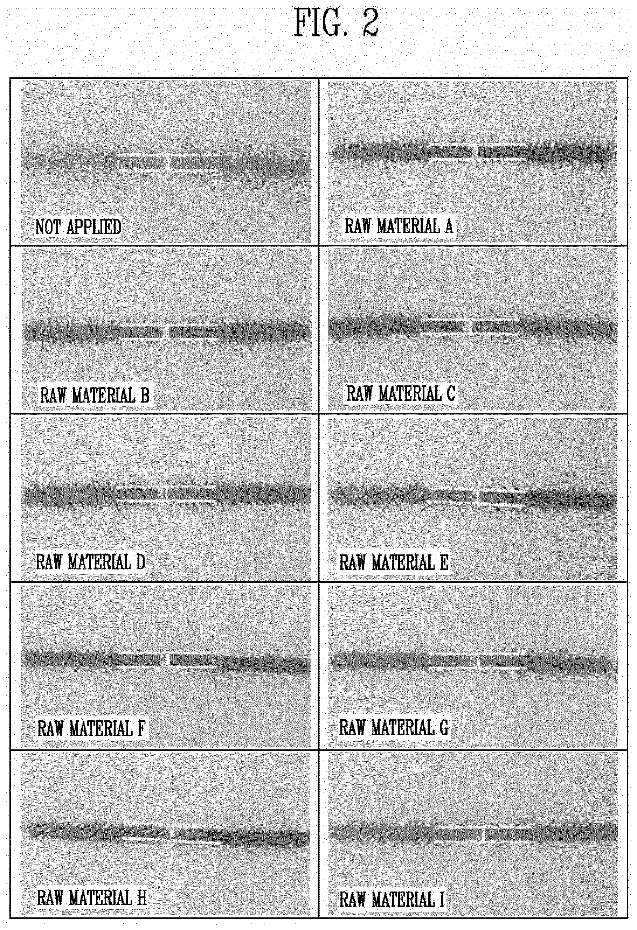

The actual skin surface was observed as shown in the photograph in FIG. 2 below.

When the raw material (raw materials A to I) used in the ink fixer composition for tattoo printing of the present invention was first applied to a skin and then an ink is printed, it was confirmed that the ink did not easily smudge along the skin wrinkles and the ink smudging phenomenon was clearly controlled compared to when the ink was printed without applying the ink fixer composition (not applied).

In particular, when a plate-shaped raw material (raw materials A to E) was used compared to when a spherical raw material (raw materials F to I) having a spherical shape were used, there was a disadvantage in that the white turbidity phenomenon occurred frequently and the application area was conspicuous due to that, and it was confirmed that the plate-shaped raw materials have a relatively high haze value and low straight transmittance compared to the spherical raw materials. In addition, in terms of the quality of the printed image, it could be seen that the spherical raw materials exhibited a better smudging alleviation effect.

### Experimental Example 2: Confirmation of smudging alleviation effect according to the size of raw materials of the ink fixer composition for tattoo printing

The particle size, oil absorption amount, and water absorption amount of the spherical raw materials were measured and compared with each other.

Raw materials F to L were applied using a Rubycell puff to the forearm area, and an ink was printed on the raw material application area using a skin printing device. The same ink as that used in Experimental Example 1 was used for printing. The degree of smudging occurrence at the printing boundary was observed to evaluate the smudging alleviation effect.

Smudging alleviation evaluation method: ⊚: No ink smudging when observed visually; ∘: Ink smudged into fine wrinkles with a clear boundary when observed visually; △: Ambiguous boundary due to ink smudging when observed visually; and -: No effect.

As shown in Table 2 below, the smudging alleviation effect according to the size of the raw materials was confirmed.

**[Table 2]**

| **Raw materia l** | **Component** | **Manufacture r** | **Type** | **Averag e particl e size (µm)** | **Oil absorptio n (ml/g)** | **Water absorp tion (ml/g)** | **Smud ging allevia tion effect** |
|---|---|---|---|---|---|---|---|
| F | Silica, methicone | ASAHI GLASS COMPANY | Spher ical | 5 | 2.5-3 | - | ○ |
| G | Silica | ASAHI GLASS COMPANY | | 5 | 3-4 | 2.5-2.8 | ⊚ |
| H | Silica | SUNJIN CHEMICAL | | 7 | 0.9-1.3 | 0.9-1.1 | ○ |
| I | Silica | ABC NANOTECH | | 9 | 1.5 | 1.3-1.5 | ○ |
| J | Polymethyl methacrylate | SUNJIN CHEMICAL | | 7 | 0.45 | 0.4-0.7 | ○ |
| K | methyl methacrylate crosspolymer | SUNJIN CHEMICAL | | 8 | 1.6-1.8 | - | ○ |
| L | vinyl dimethicone/methicone silsesquioxane crosspolymer | SHINETSU | | 12 | Selective oil absorption amount | - | Δ |

When the particle size of the raw material exceeded 12 µm, the smudging alleviation effect was poor, and when the particle size of the raw material was 5 to 9 µm, the smudging alleviation effect was excellent. However, the smudging alleviation effect according to the particle size did not differ significantly when the particle size was between 5 to 9 µm.

In addition, it was confirmed that when the particle size was within 5 to 9 µm, the smudging alleviation effect was excellent as a sum of a water absorption amount and an oil absorption amount was large. It was confirmed that the smudging alleviation effect was the best when both the oil absorption amount and the water absorption amount were large, and the smudging alleviation effect was good when either the oil absorption amount or the water absorption amount was large. Therefore, it is preferable that a raw material has both a high oil absorption amount and a high water absorption amount, and specifically, it is preferable that a raw material of an ink fixer composition for tattoo printing has a sum of an oil absorption amount and a water absorption amount of 0.8 ml/g or more.

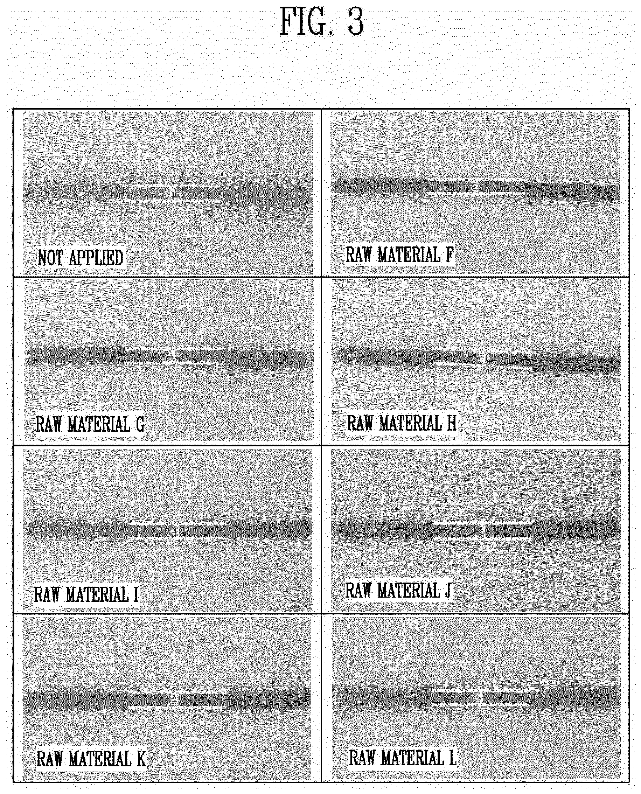

The actual skin surface was observed as shown in the photograph in FIG. 3 below.

When raw materials F and G that had similar sizes were compared, raw material G, which has a high water absorption amount compared to raw material F, which has a hydrophobic coating on the raw material powder surface and thus has no absorption amount, exhibited a superior smudging alleviation effect.

When raw materials G to I composed of the same silica were compared, raw material G, which has a small size and excellent oil absorption amount and water absorption amount, exhibited a superior smudging alleviation effect.

### Experimental Example 3: Confirmation of performance (smudging alleviation, usability, transparency) according to the blending ratio of the ink fixer composition for tattoo printing

Each component of an ink fixer composition for tattoo printing prepared according to the present invention was blended as shown in Table 3 below.

**[Table 3]**

| **Component** | **Example 1** | **Example 2** | **Example 3** | **Example 4** | **Comparative Example 1** | **Comparative Example 2** |
|---|---|---|---|---|---|---|
| **Synthetic fluorophlogopite** | 5 | 30 | 10 | 20 | 0 | 40 |
| **Alumina** | 25 | 0 | 0 | 0 | 0 | 0 |
| **Silica (raw material I)** | 4 | 4 | 4 | 4 | 4 | 4 |
| **Silica (raw material G)** | 60 | 60 | 80 | 70 | 90 | 50 |
| **1,2-Hexanediol** | 1 | 1 | 1 | 1 | 1 | 1 |
| **Hexyl laurate** | 5 | 5 | 5 | 5 | 5 | 5 |
| **Total (% by weight)** | 100 | 100 | 100 | 100 | 100 | 100 |

The powder part of each example and comparative example composition was placed in a 5 L Henschel mixer equipped with an impeller and stirred at 2,000 rpm and 1,000 rpm for five minutes while spraying the oil binder part. After spraying all of the oil binder part, the oil binder part was mixed at 2,000 rpm for three minutes so that it could be evenly mixed with the powder part, and then a pulverizing process was performed to improve the dispersion of the oil binder part, and the resulting product was filtered through a 50-mesh net. Thereafter, a maturing process was performed by allowing the resulting product to stand for 24 hours to remove uneven pores in the mixture.

The smudging alleviation effect of each example and comparative example composition was observed by printing with a skin printing device and observing the degree of smudging that occurred at the printing boundary. The ink used for printing was the same ink as the ink used in Experimental Example 1. In addition, a blind test was conducted with 10 women aged between 20 and 40 to perform sensory evaluation of the powdering when applied, the skin adhesion, and the transparency.

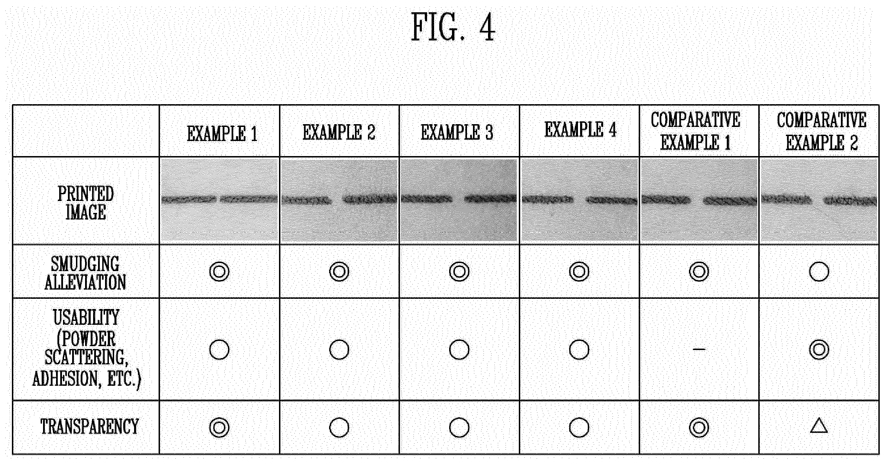

The smudging alleviation effect, usability, and transparency of Examples 1 to 4 and Comparative Examples 1 and 2 were evaluated, and the results are shown in FIG. 4 below.

Smudging alleviation evaluation method: ⊚: No ink smudging when observed visually; ∘: Ink smudged into fine wrinkles with a clear boundary when observed visually; △: Ambiguous boundary due to ink smudging when observed visually; and -: No effect.

Usability evaluation method: ⊚: No powder scattering observed visually when applied; ∘: Powder scattering observed visually when applied but adhered to the skin; △: Powder scattering observed visually when applied and poorly adhered to the skin; -: Significant powder scattering observed visually, making it difficult to use.

Transparency evaluation method: ⊚: The skin's natural color shown when visually observed; ∘: Faint whiteness confirmed on the skin when visually observed; △: Whiteness confirmed on the skin when visually observed; -: Foreign whiteness confirmed on the skin when visually observed.

As the results shown in FIG. 4, Examples 1 to 4 and Comparative Example 1, in which raw material G with excellent water absorption and oil absorption a relatively small size was applied in an amount of 60% or more, exhibited the best performance in smudging alleviation, and Comparative Example 1, which had a low amount of plate-like powder, and Example 1, which contained alumina, exhibited the best performance in transparency. When Examples 2 to 4 and Comparative Example 2 were compared, the higher the amount of plate-like powder in the composition, the more the white turbidity of the composition and the lower the transparency, so a composition including excessive plate-like powder was not suitable for use as an ink fixer. However, when Examples 1 to 4 and Comparative Example 1 were compared, the usability of the composition could be reduced when plate-like powder was not added. Therefore, it is preferable that plate-like powder is applied in an amount below an amount level in the composition to increase usability without significantly affecting the smudging alleviation effect.

Each component of an ink fixer composition for tattoo printing prepared according to the present invention was blended as shown in Table 4 below.

**[Table 4]**

| **Component** | **Example 1** | **Example 5** | **Comparative Example 3** |
|---|---|---|---|
| **Synthetic fluorophlogopite** | 5 | 5 | 5 |
| **Alumina** | 25 | 25 | 25 |
| **Silica (raw material I)** | 4 | 34 | 54 |
| **Silica (raw material G)** | 60 | 30 | 10 |
| **1,2-Hexanediol** | 1 | 1 | 1 |
| **Hexyl laurate** | 5 | 5 | 5 |
| **Total (% by weight)** | 100 | 100 | 100 |

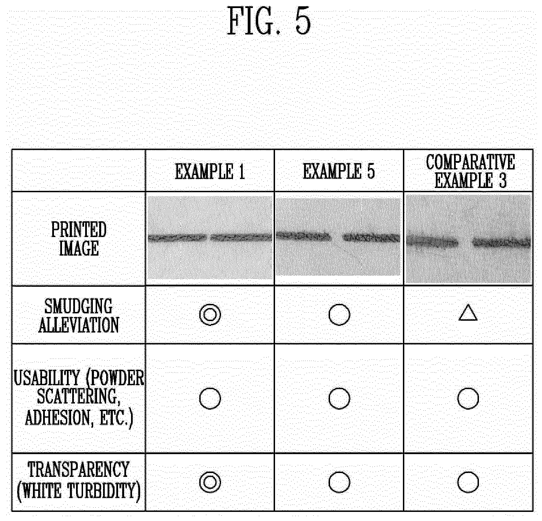

The smudging alleviation effect, usability, and transparency of Examples 1, 5, and Comparative Example 3 were evaluated, and the results are shown in FIG. 5 below. The same ink as the ink used in Experimental Example 1 was used for printing.

Smudging alleviation evaluation method: ⊚: No ink smudging when observed visually; ∘: Ink smudged into fine wrinkles with a clear boundary when observed visually; △: Ambiguous boundary due to ink smudging when observed visually; and -: No effect.

Usability evaluation method: ⊚: No powder scattering observed visually when applied; ∘: Powder scattering observed visually when applied but adhered to the skin; △: Powder scattering observed visually when applied and poorly adhered to the skin; -: Significant powder scattering observed visually, making it difficult to use.

Transparency evaluation method: ⊚: The skin's natural color shown when visually observed; ∘: Faint whiteness confirmed on the skin when visually observed; △: Whiteness confirmed on the skin when visually observed; -: Foreign whiteness confirmed on the skin when visually observed.

As shown in FIG. 5, when raw material I was applied in a high amount, some smudging occurred and a satisfactory smudging alleviation effect was not achieved.

Considering that the smudging alleviation effect was excellent as the water absorption amount and the oil absorption amount in Table 2 are large, it is preferable to use silica having a large sum of an oil absorption amount and a water absorption amount, and considering that the sum of the oil absorption amount and the water absorption amount of silica (raw material G) was 5.5 to 6.8 ml/g and the sum of the oil absorption amount and the water absorption amount of silica (raw material I) was 2.8 to 3.0 together with the results shown in Table 8, it is preferable that an ink fixer composition for tattoo printing according to the present invention includes spherical powder having a sum of an oil absorption amount and a water absorption amount of 4 ml/g or more, preferably 5 ml/g or more, in an amount of 16% or more, 26% or more, 36% or more, or 46% or more, based on the total weight of the spherical powder.

Each component of an ink fixer composition for tattoo printing prepared according to the present invention was blended as shown in Table 5 below.

**[Table 5]**

| **Component** | **Example 1** | **Example 6** | **Comparative Example 4** | **Comparative Example 5** |
|---|---|---|---|---|
| **Synthetic fluorophlogopite** | 5 | 5 | 5 | 5 |
| **Alumina** | | | | |
| **Silica (raw material I)** | 25 | 25 | 25 | 25 |
| **Silica (raw material G)** | 4 | 4 | 4 | 4 |
| **1,2-Hexanediol** | 60 | 55 | 45 | 65 |
| **Hexyl laurate** | 1 | 1 | 1 | 1 |
| **Total (% by weight)** | 5 | 10 | 20 | 0 |
| | 100 | 100 | 100 | 100 |

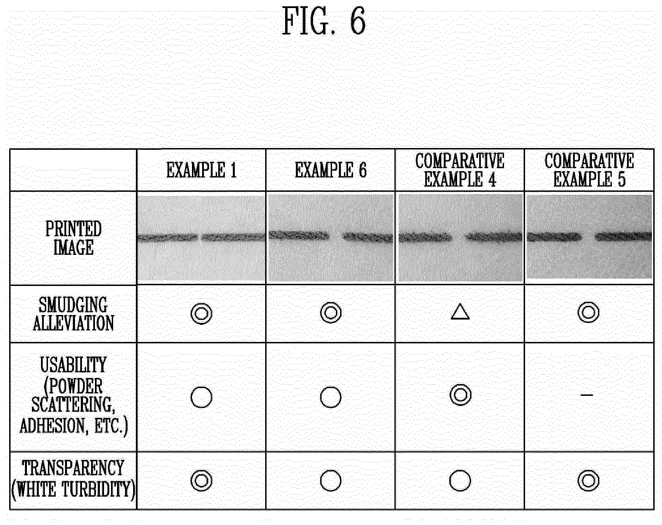

The smudging alleviation effect, usability, and transparency of Examples 1 and 6 and Comparative Examples 4 and 5 were evaluated, and the results are shown in FIG. 6 below. The same ink as the ink used in Experimental Example 1 was used for printing.

Smudging alleviation evaluation method: ⊚: No ink smudging when observed visually; ∘: Ink smudged into fine wrinkles with a clear boundary when observed visually; △: Ambiguous boundary due to ink smudging when observed visually; and -: No effect.

Usability evaluation method: ⊚: No powder scattering observed visually when applied; ∘: Powder scattering observed visually when applied but adhered to the skin; △: Powder scattering observed visually when applied and poorly adhered to the skin; -: Significant powder scattering observed visually, making it difficult to use.

Transparency evaluation method: ⊚: The skin's natural color shown when visually observed; ∘: Faint whiteness confirmed on the skin when visually observed; △: Whiteness confirmed on the skin when visually observed; -: Foreign whiteness confirmed on the skin when visually observed.

Hexyl laurate was used as a binder to control the adhesion and usability of the composition. The binder is preferably 3% to 15% by weight, more preferably 5% to 10% by weight, of the total weight of the composition, as shown in FIG. 6.

Experimental Example 4: Confirmation of skin irritation of a solid ink fixer composition for tattoo printing compared to a liquid composition

A liquid ink fixer composition for tattoo printing must be dried on a skin to serve as a fixer on the skin. At this time, ethanol and a film-forming agent may be added to the liquid ink fixer composition to help dry the liquid ink fixer composition for tattoo printing.

Experimental Example 4-1: The drying speed and skin irritation of the composition according to the amount of ethanol were evaluated by the following experimental method.
1) A liquid ink fixer composition for tattoo printing including 10% of a film-forming agent (film-forming agent: Methacryloyl Ethyl Betaine/Acrylates Copolymer) is prepared by varying the amount of ethanol as shown in Table 6 below.

**[Table 6]**

| **Raw material** | **Liquid 1-1** | **Liquid 1-2** | **Liquid 1-3** | **Liquid 1-4** | **Liquid 1-5** |
|---|---|---|---|---|---|
| **Purified water** | 80 | 60 | 40 | 20 | 0 |
| **Ethanol** | 10 | 30 | 50 | 70 | 90 |
| **Film-forming agent** | 10 | 10 | 10 | 10 | 10 |

2) Under conditions of 24.5 °C and 30% humidity, one drop of the liquid ink fixer composition is applied to a 1x2 cm rectangular area of the skin and the drying time is measured.
3) Skin irritation (skin pulling, stinging, redness, etc.) is evaluated after drying.

Irritation evaluation method: -: No abnormality, +: Mild stinging/itching/redness, ++: Moderate stinging/itching/redness, +++: Severe stinging/itching/redness.

As shown in Table 7 below, as the amount of ethanol in the liquid ink fixer composition for tattoo printing increased, the drying speed of the composition increased, but the skin irritation of the liquid ink fixer composition increased.

**[Table 7]**

| **Raw material** | **Liquid 1-1** | **Liquid 1-2** | **Liquid 1-3** | **Liquid 1-4** | **Liquid 1-5** |
|---|---|---|---|---|---|
| **Drying speed (sec)** | 200 | 150 | 80 | 50 | 20 |
| **Skin irritation** | + | + | ++ | +++ | +++ |

Experimental Example 4-2: The skin irritation and cleansing easiness of the ink fixer composition according to the film-forming agent content were evaluated by the following experimental method.
1) A liquid ink fixing agent composition for tattoo printing was prepared by varying the amount of the film-forming agent as shown in Table 8 below.

**[Table 8]**

| **Raw material** | **Liquid 2-1** | **Liquid 2-2** | **Liquid 2-3** | **Liquid 2-4** | **Liquid 2-5** |
|---|---|---|---|---|---|
| **Purified water** | 50 | 45 | 40 | 35 | 30 |
| **Ethanol** | 50 | 50 | 50 | 50 | 50 |
| **Film-forming agent** | 0 | 5 | 10 | 15 | 20 |

2) Under conditions of 24.5 °C and 30% humidity, one drop of the liquid ink fixer composition is applied to a 1x2 cm rectangular area of the skin and the drying time is measured.
3) After drying, skin irritation (skin pulling, stinging, redness, etc.) and cleansing easiness (whether the ink fixer composition is easily removed from the skin) are evaluated.
- Irritation evaluation method: -: No abnormality, +: Mild stinging/itching/redness, ++: Moderate stinging/itching/redness, +++: Severe stinging/itching/redness.
- Cleansing evaluation method: +++: Removable with lukewarm water, ++: Removable by rubbing with a cleansing foam 30 times, +: Removable by rubbing with a cleansing foam 60 times, -: Residue remaining even after rubbing with a cleansing foam 100 times.

As shown in Table 9 below, as the amount of the film-forming agent in the liquid ink fixer composition for tattoo printing increased, the skin irritation of the liquid ink fixer composition increased and cleansing became more difficult.

**[Table 9]**

| **Raw material** | **Liquid 2-1** | **Liquid 2-2** | **Liquid 2-3** | **Liquid 2-4** | **Liquid 2-5** |
|---|---|---|---|---|---|
| **Skin irritation** | + | ++ | ++ | +++ | +++ |
| **Cleaning easiness** | +++ | ++ | + | - | - |

Experimental Example 4-3: The skin irritation and cleansing easiness of the solid ink fixer composition according to the film-forming agent content were evaluated by the following experimental method.
1) Under conditions of 24.5 °C and 30% humidity, the solid ink fixer composition is applied to a 1x2 cm rectangular area of the skin.
2) Skin irritation (skin pulling, stinging, redness, etc.) and cleansing easiness (whether the ink fixer composition is easily removed from the skin) are evaluated.
   - Irritation evaluation method: -: No abnormality, +: Mild stinging/itching/redness, ++: Moderate stinging/itching/redness, +++: Severe stinging/itching/redness.
   - Cleansing evaluation method: +++: Removable with lukewarm water, ++: Removable by rubbing with a cleansing foam 30 times, +: Removable by rubbing with a cleansing foam 60 times, -: Residue remaining even after rubbing with a cleansing foam 100 times.

As shown in Table 10 below, it was confirmed that the solid ink fixer composition exhibited no skin irritation and showed excellent cleansing properties (stick type: a formulation prepared by solidifying in water using sodium stearate as an aqueous hardness forming agent; powder type: a formulation homogenized by pulverizing absorbent silica).

**[Table 10]**

| | **Solid 1 (powder type)** | **Solid 2 (stick type)** |
|---|---|---|
| **Skin irritation** | - | - |
| **Cleaning easiness** | +++ | +++ |

In this way, the solid ink fixer composition is fixed immediately upon application, and does not irritate the skin because no additional component such as ethanol or a film-forming agent is added. In addition, the solid ink fixer composition is easy to cleanse compared to the liquid ink fixing agent composition, so users can use it conveniently.

### Experimental Example 5: Confirmation of skin surface uniformizing effect of the solid ink fixer composition for tattoo printing compared to the liquid composition

The uniformizing effect of the ink fixer composition for tattoo printing was evaluated by the following experimental method.
1) The roughness (Ra) of the artificial skin surface was measured five times using the ANTERA 3D device, and then the average value was derived. The unit of roughness is an arbitrary unit.
2) The liquid and solid ink fixer compositions for tattoo printing were each applied to the artificial skin, and the roughness was measured (Liquid 1-3 was used as the liquid ink fixer composition, and powder type, and the stick type were used as the solid ink fixer composition.).
3) The skin surface uniformizing effect of the ink fixer compositions was compared by comparing the roughness values.

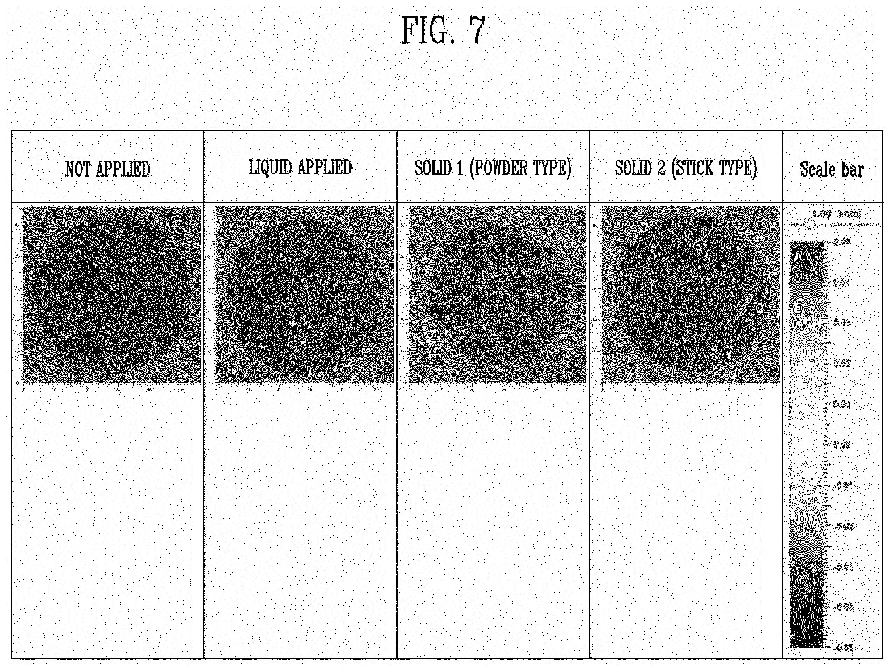

In FIG. 7 below, the average surface height of the entire skin image was set to 0, and the surface height between +0.05 mm (red) and -0.05 mm (purple) was expressed in colors according to the scale bar. The more red and purple areas there are, the greater the difference in the surface height according to positions, which indicates greater roughness.

As shown in FIG. 7, it was confirmed that regardless of the state of the ink fixer composition, that the ink fixer composition can uniformize the skin when applied, compared to when it is not applied. When the liquid ink fixer composition and the solid ink fixer composition were compared, the red and purple areas were less when the solid ink fixer composition was applied compared to when the liquid fixer composition was applied, and accordingly, it was confirmed that the solid ink fixer composition can make the skin more uniform.

FIG. 1 shows a graph illustrating the roughness values of the artificial skin surface according to the type of the ink fixer composition for tattoo printing.

Referring to FIG. 1, the roughness value was 29.6 when the ink fixer composition was not applied to the skin (not applied), and 27.4 when the liquid ink fixer composition was applied to the skin. When the solid ink fixer composition was applied to the skin, the powder type exhibited a roughness value of 24.4, and the stick type exhibited a roughness value of 22.8.

The skin roughness value was smaller when the ink fixer composition was applied than when the ink fixer composition was not applied, and the skin roughness value was smaller when the solid ink fixer composition was applied than when the liquid ink fixer composition was applied. A smaller roughness value indicates a more uniform skin surface, so regardless of the state of the ink fixer composition, the ink fixer composition can make the skin uniform when applied compared to when not applied. In addition, when the liquid ink fixer composition and the solid ink fixer composition are compared, the solid ink fixer composition can make the skin uniform compared to the liquid ink fixer composition.

### Experimental Example 6-1: Solidification suppression performance of the ink composition according to humectant type and content

The state of the ink composition after drying was confirmed according to the type and content of humectant included in the ink composition for tattoo printing.

The ink composition contained 5% by weight of a colorant (Yellow No. 4, manufacturer: Bolak CO., LTD.) and contained a humectant as shown in Table 11 below, and purified water added to fill up the remaining content except for the humectant (to 100% by weight). Each ink composition was divided into a certain amount and allowed to stand overnight in a high-temperature chamber at 50 °C, and the state of each ink composition was observed.

Solidification inhibition evaluation method: ∘: Completely liquid form; △: Partial suppression of solidification confirmed; X: Completely solid form.

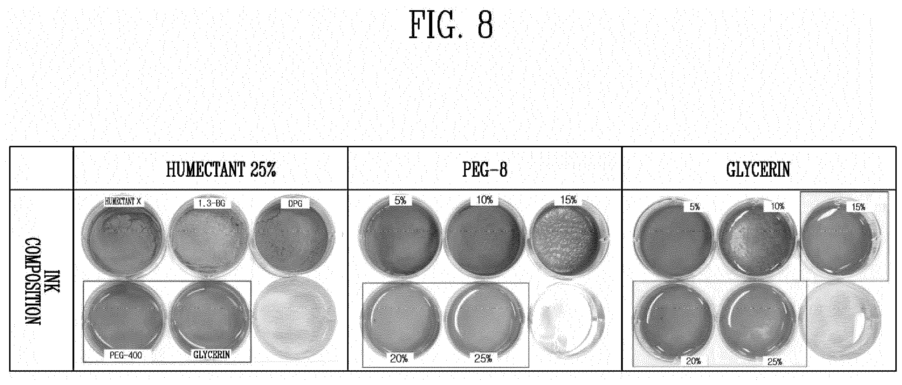

As shown in Table 11 below and FIG. 8, the ink composition was solidified even when it contained 25% by weight or more of dipropylene glycol (DPG) and 1,3-butylene glycol (1,3-BG), whereas the solidification of the ink composition was suppressed when it contained 15% by weight or more of PEG-8 or 10% by weight or more of glycerin. The ink composition did not solidify at all when it contained 20% by weight or more of PEG-8 or 15% by weight or more of glycerin.

**[Table 11]**

| **Humectant** | **Manufacturer** | **0%** | **5%** | **10%** | **15%** | **20%** | **25%** |
|---|---|---|---|---|---|---|---|
| **Dipropylene glycol** | SKC CO., LTD. | X | X | X | X | X | △ |
| **1,3-Butylene glycol** | OXEA | X | X | X | X | X | △ |
| **PEG-8** | SURFACHEM | X | X | X | △ | ○ | ○ |
| **Glycerin** | LG H&H | X | X | △ | ○ | ○ | ○ |

Specifically, FIG. 8 below shows the results obtained from the ink compositions containing 25% of a humectant according to the type of the humectant (humectant 25%), from the ink compositions containing PEG-8 according to the content of the humectant (PEG-8), and the ink compositions containing glycerin according to the content of the humectant (glycerin).

According to Table 11 and FIG. 8 above, an ink composition for tattoo printing according to one embodiment of the present invention preferably includes a humectant in an amount of 10% to 40% by weight, or 15% to 30% by weight, based on the total weight percentage of the ink composition, and more preferably, it may include PEG-8 in an amount of 15% to 40% by weight, or 15% to 30% by weight; or it may include glycerin 10% to 40% by weight, or 10% to 30% by weight.

### Experimental Example 6-2: Solidification suppression performance of the ink compositions according to the content of a humectant prepared by mixing PEG-8 and glycerin

The ink compositions contained 5% by weight of a colorant (Yellow No. 4, manufacturer: Bolak CO., LTD.), and a humectant, prepared by mixing PEG-8 and glycerin was used as shown in Table 12 below. Purified water added to fill up the remaining content except for the humectant (to 100% by weight). Each ink composition was divided into a certain amount and allowed to stand overnight in a 50 °C high-temperature chamber, and the state of each ink composition was observed.

Solidification inhibition evaluation method: ∘: Completely liquid form; △: Partial suppression of solidification confirmed; X: Completely solid form.

The ink composition for tattoo printing according to one embodiment of the present invention preferably includes 15% to 40% by weight, or 20% to 30% by weight, of a mixed humectant of PEG-8 and glycerin based on the total weight percentage of the ink composition.

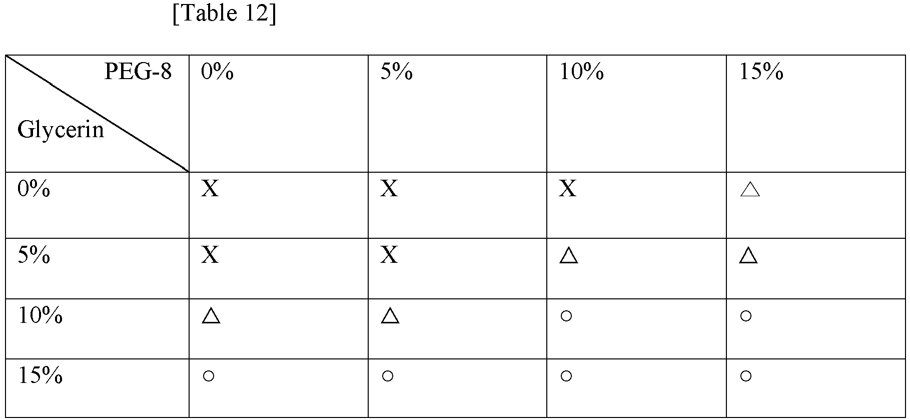

### Experimental Example 7: Evaluation of the impregnation properties of ink compositions into ink storage sponge according to the type of surfactant

Meanwhile, an ink composition is generally stored in a cartridge, and a sponge for the purpose of retaining the ink is provided in the cartridge. At this time, the sponge is impregnated with the ink to prevent the ink from leaking out of the cartridge, and the ink is appropriately supplied to the nozzle during printing. The sponge impregnated with an ink may be mainly made of polyurethane, and polyurethane foam may have excellent ink retaining ability due to its porous structure with uniform porosity.

When the impregnation properties of an ink composition into a sponge is low, there is a problem that it takes a long time to fill the ink composition into the cartridge or sponge, so it is necessary to improve the impregnation properties of the ink composition to solve this problem.

To stably and continuously discharge an ink composition, the ink composition should be well impregnated into a sponge, and no bubbles should be generated in the ink composition. To improve the impregnation properties of the ink composition for the sponge and reduce the amount of bubbles generated, it is preferable that the ink composition includes a surfactant. As described below, the sponge impregnation of the ink compositions according to the type of surfactant was confirmed.

As shown in Table 13 below, the sponge impregnation was evaluated by dropping 5 µl of each surfactant onto a polyurethane sponge (HP62 empty (3UB14A) inner sponge) using a micropipette, and measuring the time it took for each surfactant to be absorbed (Impregnation: ⊚ = Immediately absorbed, ∘ = Absorbed within 10 minutes, △ = Absorbed within one hour, X = Not absorbed, HLB: Hydrophile-Lipophile Balance).

It was confirmed that among the surfactants, polyglyceryl-4 caprate and PEG-7 glyceryl cocoate were confirmed to be rapidly absorbed into the sponge and exhibit excellent impregnation.

**[Table 13]**

| **Component** | **Manufacturer** | **HLB** | **Impregnation properties** |
|---|---|---|---|
| GLYCERETH-26 | - | 18 | △ |
| Polysorbate 20 | - | 16.7 | △ |
| PEG-60 GLYCERYL ISOSTEARATE | NIHON EMULSION | 16 | △ |
| POLYGLYCERYL-4 CAPRATE | EVONIC | 16 | ⊚ |
| POLYGLYCERYL-10 OLEATE, POLYGLYCERYL-10 STEARATE | BIOBEAUTECH | 15.1 | △ |
| PEG-60 Hydrogenated castor oil | Croda | 14.7 | △ |
| PEG-40 Hydrogenated castor oil | - | 13 | △ |
| PEG-7 GLYCERYL COCOATE | BASF | 11 | ⊚ |
| PEG-8 GLYCERYL ISOSTEARATE | NIHON EMULSION | 10 | △ |
| PEG-20 GLYCERYL TRIISOSTEARATE | NIHON OILLIO | 8 | △ |

The ink composition may include components as shown in Table 14 below, and may include PEG-7 glyceryl cocoate and polyglyceryl-4 caprate as surfactants. It was confirmed that when an ink composition includes PEG-7 glyceryl cocoate and polyglyceryl-4 caprate as surfactants, the impregnation properties were excellent. The ink composition according to the present invention preferably includes 0.001% to 10% by weight, preferably 0.01% to 5% by weight, of PEG-7 glyceryl cocoate or polyglyceryl-4 caprate based on the total weight of the ink composition.

**[Table 14]**

| **Component** | **Ink composition 1** | **Ink composition 2** |
|---|---|---|
| Purified water | 66.5 | 66.5 |
| 1,2-hexanediol | 3 | 3 |
| Glycerin | 15 | 15 |
| PEG-8 | 10 | 10 |
| PEG-7 glyceryl cocoate | 0.5 | |
| Polyglyceryl-4 caprate | | 0.5 |
| Yellow No. 4 | 5 | 5 |
| Impregnation properties | ⊚ | ⊚ |

### Experimental Example 8: Evaluation of bubble generation suppression performance of ink compositions according to the type and content of antifoamer

Meanwhile, since bubbles (air) are dispersed in an ink composition, it is preferable that an antifoamer (defoaming agent) that prevents bubble formation or destroys bubbles is included in the ink composition to eliminate the bubbles. When an antifoamer is included, bubble formation in an ink composition is suppressed and the discharge stability of the ink composition is improved.

In addition, it is preferable that an antifoamer included in an ink composition has selective miscibility. This is because when an antifoamer has good compatibility with an ink composition, the antifoamer does not move to the bubble film but mixes with the ink composition, which weakens the antifoaming effect, and when the compatibility is not good, the antifoamer may cause a haze phenomenon in the ink composition and may cause a printing defect problem of the ink composition. Accordingly, an antifoamer included in an ink composition must have appropriate compatibility with the ink composition.

Particulate silicone antifoamers commonly used in cosmetics may clog nozzles when used in ink compositions, and are not very compatible with ink compositions for tattoo printing, so particulate silicone antifoamers are not suitable for use in tattoo printing ink compositions. In addition, silicone is not a preferable component to use because human health hazard and environmental pollution issues have recently been raised.

In addition, ethanol-based antifoamers are mainly used as antifoamers in cosmetics, but since ethanol is volatile, it may cause nozzle clogging when included in an ink composition, making it an unsuitable component to use.

As described below, the amount of bubbles generated when shaking ink compositions including each antifoamer according to the type and content of the antifoamer was confirmed. As shown in Table 15 below, it was confirmed that when glycereth-26 (polyethylene glycol ether composed of 26 moles of ethylene oxide and glycerin) was included, the amount of bubbles generated was reduced compared to the ink compositions (ink composition 3) that included no glycereth-26.

**[Table 15]**

| **Component** | **Ink composition 3** | **Ink composition 4** | **Ink composition 5** |
|---|---|---|---|
| **Purified water** | 66.7 | 66.6 | 66.6 |
| **1,2-hexanediol** | 3 | 3 | 3 |
| **Glycerin** | 15 | 15 | 15 |
| **PEG-8** | 10 | 10 | 10 |
| **Glycereth-26** | - | **0.1** | **0.1** |
| **PEG-7 glyceryl cocoate** | 0.5 | 0.5 | - |
| **Polyglyceryl-4 caprate** | - | - | 0.5 |
| **Yellow No. 4** | 5 | 5 | 5 |
| **Amount of bubbles produced** | **Much** | **Little** | **Little** |

An ink composition according to one embodiment of the present invention may include an antifoamer to suppress the generation of bubbles and quickly remove the generated bubbles. An ink composition according to the present invention preferably includes an antifoamer in an amount of 0.001% to 10% by weight, preferably 0.01% to 5% by weight, based on the total weight of the ink composition.

### Experimental Example 9: Evaluation of bubble removal performance of ink compositions according to plant-derived oils

As described below, when plant-derived oils (hydrogenated ethylhexyl olivate and hydrogenated olive oil unsaponifiable, viscosity: 20 cs (Spindle LV1, speed: 60 rpm, temperature 20 ± 0.2 °C), surface tension: 31.5 dyne/cm) were added as an antifoamer to the ink composition, the degree of bubble generation when the ink composition containing each surfactant was shaken and the degree of bubble removal over time were confirmed.

**[Table 16]**

| **Component** | **Ink compositi on 6** | **Ink compositi on 7** | **Ink compositi on 8** | **Ink compositi on 9** | **Ink compositi on 10** |
|---|---|---|---|---|---|
| **Purified water** | 66.55 | 66.5 | 66.4 | 66.6 | 66.7 |
| **1,2-hexanediol** | 3 | 3 | 3 | 3 | 3 |
| **Glycerin** | 15 | 15 | 15 | 15 | 15 |
| **PEG-8** | 10 | 10 | 10 | 10 | 10 |
| **Glycereth-26** | 0.1 | 0.1 | 0.1 | 0.1 | 0.1 |
| **PEG-7 glyceryl cocoate** | 0.3 | 0.3 | 0.3 | 0.2 | 0.1 |
| **Hydrogenated ethylhexyl olivate and hydrogenated olive oil unsaponifiable** | **0.05** | **0.1** | **0.2** | **0.1** | **0.1** |
| **Yellow No. 4** | 5 | 5 | 5 | 5 | 5 |
| **Amount of bubbles produced** | **Much** | **Little** | **Little** | **Little** | **Little** |
| **Amount of bubbles remaining after four hours** | **Much** | **Little** | **Very little** | **Very little** | **Little** |

Plant-derived oils may have a similar antifoaming effect to silicone (dimethicone viscosity: 100 cs (Spindle LV1, speed 60 rpm, temperature 20 ± 0.2 °C), surface tension: 21.5 dyne/cm) and may have a surface tension of 50 dyne/cm or less.

An ink composition according to the present invention may include a plant-derived oil as an antifoamer, preferably may include one of hydrogenated ethylhexyl olivate and hydrogenated olive oil unsaponifiable. A plant-derived oil may be included preferably in an amount of 0.1% to 10% by weight, preferably 0.1% to 5% by weight, and most preferably 0.1% to 2% by weight, based on the total weight of the ink composition.

### Experimental Example 10: Evaluation of image clarity according to the type and content of surfactant

An ink composition is discharged in the form of droplets from the outlet of the nozzle. An ink composition is delivered to a specific position in the form of droplets, and printing occurs at the position. In the cases where the nozzle moves at a high speed and discharges the ink composition, when the time for the droplets to be formed becomes longer and the length of the droplet increases, while the nozzle moves, the ink droplets may not be printed only at a specific position, and the tail of the droplets may affect the printing at other positions. When the ink droplets affect the printing at other positions, the clarity of the ink composition may be reduced, so the clarity may be affected by the time for the ink droplets to be formed at the nozzle outlet, the length of the droplets, and the like.

Meanwhile, when ink droplets are discharged to the outside, the higher the surface tension of the ink, the more the ink tends to reduce the surface area that comes into contact with the air and lower the surface energy, so that the ink may form the shape of the droplets in a short time. Accordingly, an ink with a high surface tension takes less time for the tail of the droplets to separate from the nozzle (breakup time), and the distance from the end of the nozzle to the tip of the droplets (breakup distance) is short. Therefore, when the surface tension of an ink is high, the volume of the droplets may be small, the speed of the droplets may be slow, and the number of satellites may be small.

When an ink is printed at a high speed, it is preferable that the breakup time and breakup distance are short, and it is also preferable that the volume of the droplets is appropriately controlled, so it is preferable that the surface tension of the ink is maintained at an appropriate value in order to maintain stable droplet discharge during ink printing.

In particular, since an water-based ink contains a large amount of water, which has a high surface tension, it is important to appropriately control the surface tension of the water-based ink, and a surface tension modifier, surfactant, or the like may be used to control the surface tension. A surfactant added to an ink composition may serve to appropriately control the surface tension of an ink composition to achieve excellent printing quality when printed.

As described below, the image clarity according to the type and content of the surfactant was evaluated. For the evaluation, ink compositions 11 to 13 were prepared as shown in Table 17 below.

**[Table 17]**

| | Ink composition 11 | | | Ink composition 12 | | | Ink composition 13 | | |
|---|---|---|---|---|---|---|---|---|---|
| **Purified water** | to 100 | to 100 | to 100 | to 100 | to 100 | to 100 | to 100 | to 100 | to 100 |
| **1,2-hexanediol** | 3 | 3 | 3 | 3 | 3 | 3 | 3 | 3 | 3 |
| **Glycerin** | 15 | 15 | 15 | 15 | 15 | 15 | 15 | 15 | 15 |
| **PEG-8** | 10 | 10 | 10 | 10 | 10 | 10 | 10 | 10 | 10 |
| **Glycereth-26** | | | | 0.1 | 0.1 | 0.1 | 0.1 | 0.1 | 0.1 |
| **PEG-7 glyceryl cocoate** | 0.5 | 0.5 | 0.5 | 0.5 | 0.5 | 0.5 | | | |
| **Polyglyceryl-4 caprate** | | | | | | | 0.5 | 0.5 | 0.5 |
| **Yellow No. 4** | 5 | | | 5 | | | 5 | | |
| **Blue No. 1** | | 5 | | | 5 | | | 5 | |
| **Red. No. 227** | | | 5 | | | 5 | | | 5 |

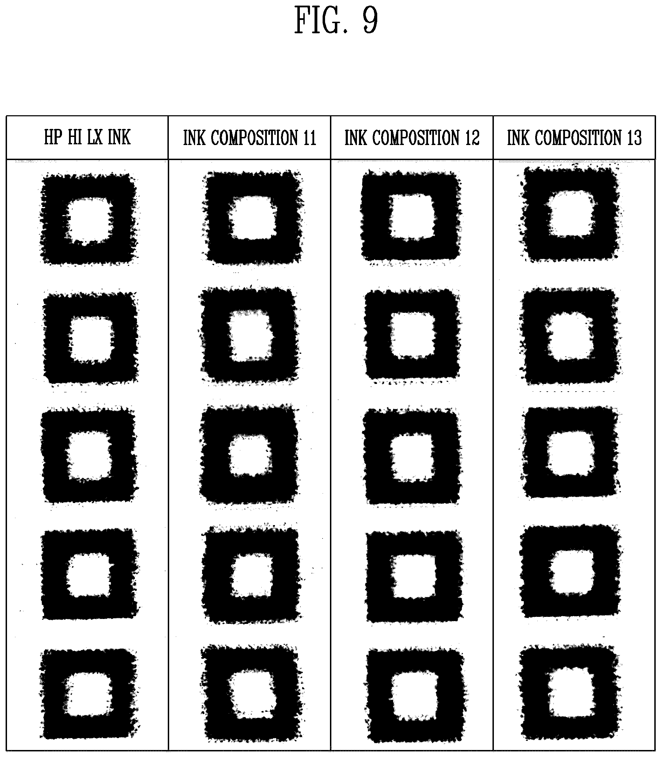

In FIG. 9 below, it was confirmed whether three colors of the ink were printed clearly when ink compositions 11 to 13 were printed on paper under the same conditions. The HP HI LX ink, a finished ink cartridge product using a cartridge container of the same specifications sold by Hewlett Packard (HP), was employed to compare the printing quality with the ink compositions of the present invention. As shown in FIG. 9 below, it was confirmed that the clarity of ink compositions 12 and 13 was as high as HP HI LX ink.

### Experimental Example 11: Evaluation of image clarity according to surfactant and antifoamer

For evaluation, ink compositions 14 to 18 were prepared as shown in Table 18 below.

**[Table 18]**

| Component | Ink composition 14 | | | Ink composition 15 | | | Ink composition 16 | | | Ink composition 17 | | | Ink composition 18 | | |
|---|---|---|---|---|---|---|---|---|---|---|---|---|---|---|---|
| **Purified water** | to 100 | to 100 | to 100 | to 100 | to 100 | to 100 | to 100 | to 100 | to 100 | to 100 | to 100 | to 100 | to 100 | to 100 | to 100 |
| **1,2-hexanediol** | 3 | 3 | 3 | 3 | 3 | 3 | 3 | 3 | 3 | 3 | 3 | 3 | 3 | 3 | 3 |
| **Glycerin** | 15 | 15 | 15 | 15 | 15 | 15 | 15 | 15 | 15 | 15 | 15 | 15 | 15 | 15 | 15 |
| **PEG-8** | 10 | 10 | 10 | 10 | 10 | 10 | 10 | 10 | 10 | 10 | 10 | 10 | 10 | 10 | 10 |
| **Glycereth-26** | 0.1 | 0.1 | 0.1 | 0.3 | 0.3 | 0.3 | 0.1 | 0.1 | 0.1 | 0.1 | 0.1 | 0.1 | 0.3 | 0.3 | 0.3 |
| **PEG-7 glyceryl cocoate** | | | | | | | 0.5 | 0.5 | 0.5 | 0.3 | 0.3 | 0.3 | 0.5 | 0.5 | 0.5 |
| **Polyglyceryl-4 caprate** | 5 | | | 5 | | | 5 | | | 5 | | | 5 | | |
| **Yellow No. 4** | | 5 | | | 5 | | | 5 | | | 5 | | | 5 | |
| **Blue No. 1** | | | 5 | | | 5 | | | 5 | | | 5 | | | 5 |

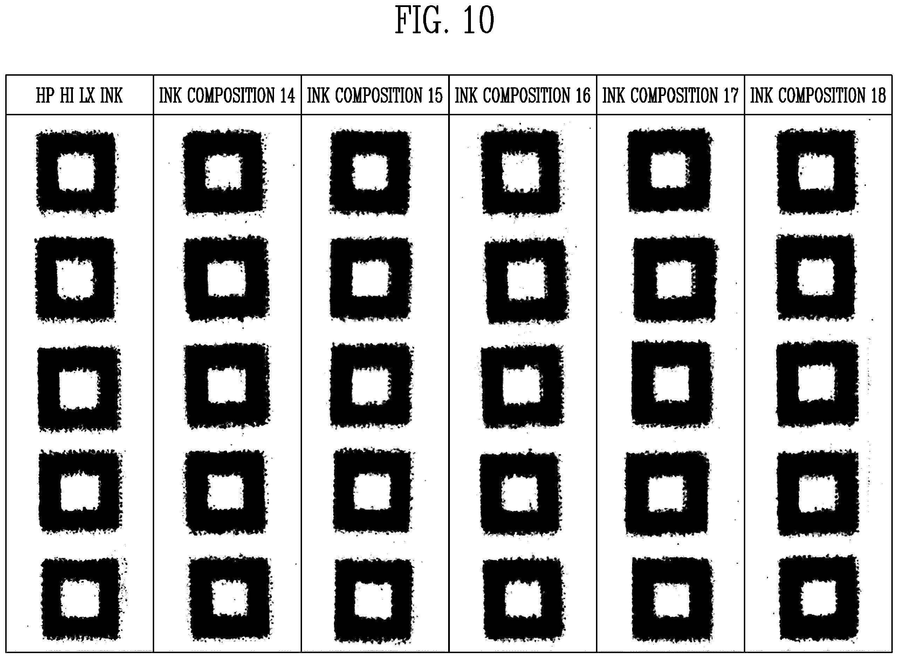

In FIG. 10 below, it was confirmed whether the three colors of ink were printed clearly when ink compositions 14 to 18 were printed on paper under the same conditions. It was confirmed that it was confirmed that the clarity of ink compositions 16, 17, and 18 was as high as HP HI LX ink, and ink composition 17 was clearer than ink composition 16.

### Experimental Example 12: Performance evaluation of fixer composition

When directly or indirectly printed onto a skin, an ink composition is difficult to infiltrate into the skin due to the structural characteristics of the skin. In addition, water-based inks for skin printing have the characteristics of low infiltration effect and long drying time, and may be dissolved when the printed substrate comes into contact with water. Therefore, an image applied using an inkjet printing method may tend to smudge or blur when rubbed against the outside, or may be dissolved and disappear when exposed to a significant amount of water-based medium (for example, when the printed article is exposed to water or comes into contact with water through other means).

To enhance the water resistance and durability of an ink on a skin, a water-soluble polymer may be added as an adhesive component when preparing the ink. However, when a nozzle of a printing device is exposed to the outside for a long time, the ink remaining in the nozzle may dry, causing nozzle clogging, or a durability problem may occur in which the printed material may peel off from the surface of the printing medium.

When a water-based ink is applied to a skin, in order to alleviate the ink composition from smudging on the skin, improve durability, and enhance water resistance, a post-processing agent (fixer, topcoat) may be additionally applied after printing.

A fixer composition is insoluble in relation to a water-based ink, is fixed for a certain period of time after being spread on a skin where the ink composition has been printed, preferably has a certain level of hardness and viscosity or higher to achieve usability so that the ink composition does not flow down, preferably forms a film of uniform thickness on the skin, and preferably has no volatility.

**In** addition, a fixer composition is preferably colorless (transparent) so that an ink composition does not affect the clarity of a printed image. In addition, it has excellent fixing ability when an ink is applied to a skin, has convenience of use as it may be applied in a simple manner, has excellent water resistance, and preferably causes no skin irritation, or the like.

For evaluation, a fixer composition was prepared as shown in Table 19 below.

**[Table 19]**

| **Component** | **Amount (% by weight)** |
|---|---|
| Petrolatum | 65-95 |
| Synthetic wax | 0.1-10 |
| Ceresin | 0.1-10 |
| Microcrystalline wax | 5-20 |
| 1,2-hexanediol | 0.1 - 5 |

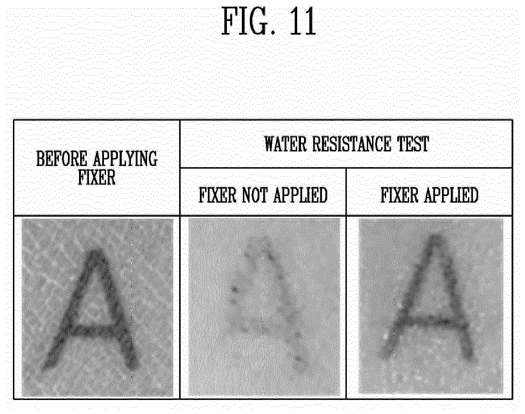

The fixer composition having the content ratio in Table 19 was applied onto a surface on which an ink composition had been applied, and the area was exposed to running water for 30 seconds. The remaining print is shown in FIG. 11 below.

The ink used was the same ink as that used in Experimental Example 1, and Example 1 of Table 5 was used as an ink fixer composition before image printing. As shown in FIG. 11, the clarity of the print was higher when a fixer was applied and then exposed to water (fixer application) than when a fixer was not applied (fixer not applied).

A fixer composition according to one aspect of the present invention may be formulated into a solid, semi-solid, a gel, or a liquid preparation, for example, a gel suitable for topical application, a water-soluble jelly, a cream, a lotion, a suspension, a solution, a foam, powder, a slurry, an ointment, a solution, an oil, a paste, a spray, an emulsion, a balm, or the like, and any formulation that may be used as a fixer composition may be used without limitation.

However, since a fixer composition should not flow off a skin in order to provide fixing ability to an ink composition, it is preferable that the fixer composition is a semi-solid formulation, and 'semi-solid' herein may mean having the consistency of a cream, an ointment, or a paste at room temperature (15 to 25 °C), and may mean a substance with no or little flowability.

As a fixer composition, it is preferable to use a balm formulation or a jelly type formulation. A balm formulation is a formulation that has a texture that is solidified into a semi-solid by mixing essential oils, glycerin, wax, and the like, and a jelly type formulation is a formulation that is solidified into a semi-solid state while containing a dispersant in a colloidal solution.

A fixer composition is insoluble in a water-based ink, and preferably an oil in a transparent, semi-solid state at room temperature. In particular, an oil-based, water-insoluble semi-solid (or paste) or solid fixer composition may immediately exhibit a water-resistant effect without a separate drying time. A hydrocarbon oil or wax is preferable as a water-insoluble semi-solid or solid fixer composition.

A fixer composition may include a hydrocarbon oil and may additionally include a hydrocarbon wax. When a hydrocarbon wax is added, water resistance may be maintained to be excellent and the spreadability and usability may be improved. A fixer composition may preferably include petrolatum, synthetic wax, ceresin, or microcrystalline wax, and when the fixer composition included synthetic wax, ceresin, and microcrystalline wax, which are hydrocarbon waxes, i) the spreading of the ink composition after printing was improved (improved fixing ability), ii) the high-temperature stability of the fixer composition was improved, and iii) the usability of the fixer composition was improved (the oiliness and stickiness reduced when applied to the skin).

In particular, petrolatum as a component is obtained from high-boiling point hydrocarbons and hydrocarbons that are usually liquid at room temperature, and is a soft, oily, semi-solid mixture of hydrocarbons, and has the characteristic of being insoluble in water, alcohol, and glycerol, and is a mixture of hydrocarbons (CₙH₂ₙ₊₂) containing only carbon and hydrogen. Petrolatum has a colorless or light yellow semi-solid form and may be utilized as a component of a fixer composition. Due to the hydrophobicity (i.e., water repellency) and water insolubility of petrolatum and the physical properties of being present in a semi-solid form at room temperature, it has excellent water resistance on a printed surface when applied alone.

A fixer composition according to the present invention preferably includes a hydrocarbon-based oil or wax in an amount of 65% to 100% by weight, preferably 65% to 95% by weight, based on the total weight percentage of the fixer composition.

As described above, an ink fixer composition for tattoo printing according to one aspect of the present invention can prevent white turbidity during application and may prevent an ink composition from smudging.

In addition, an ink fixer composition for tattoo printing according to one aspect of the present invention can prevent an ink composition from scattering in the air (flying) during the application process and can improve the skin adhesion of the ink composition.

In addition, an ink fixer composition for tattoo printing according to one aspect of the present invention is a solid ink fixer composition, which has less skin irritation and excellent cleansing easiness compared to a liquid ink fixer composition.

In addition, an ink composition for tattoo printing according to one aspect of the present invention can suppress the solidification of an ink composition by including a humectant, can reduce nozzle clogging of the ink composition, and can enable stable spraying of the ink composition.

In addition, an ink composition for tattoo printing according to one aspect of the present invention can improve the sponge impregnation properties of the ink composition by including a surfactant, and can enable stable spraying of the ink composition.

In addition, an ink composition for tattoo printing according to one aspect of the present invention can easily remove air bubbles formed in the ink composition by including an antifoamer, and can enable stable spraying of the ink composition.

In addition, an ink composition for tattoo printing according to one aspect of the present invention can control the surface tension of the ink composition by including a surfactant or an antifoamer, and thus the ink composition can provide clear printing quality.

In addition, a fixer composition for tattoo printing according to one aspect of the present invention can have a viscosity that substantially does not allow the fixer composition to flow off without an external force, and can be applied onto the surface of an area where an ink composition is applied to block the ink composition from being in contact with the outside and improve the skin fixing ability of the ink composition.

In addition, a fixer composition for tattoo printing according to one aspect of the present invention can have excellent fixing ability, high-temperature stability, and excellent usability by including a hydrocarbon-based oil or a hydrocarbon-based wax.

An ink fixer composition may be applied to a skin before an ink composition for tattoo printing according to one aspect of the present invention is printed on the skin or after the ink composition is printed on the skin.

An ink fixer or primer is preferably used before an ink composition for tattoo printing according to the present invention is printed on a skin, or a fixer or topcoat is preferably used after the ink composition is printed on the skin. A fixer may be used before an ink is printed on the skin, or an ink fixer may be used after the ink is printed on the skin. A conventional fixing agent (primer) or fixer (topcoat) known in the art may also be used before and after an ink is printed on the skin.

A tattoo printing ink fixer composition used before an ink composition is printed on a skin and a fixer composition used after the ink composition is printed on the skin may be provided.

In addition, the present invention may provide a tattoo printing kit including: an ink composition for tattoo printing; an ink fixer composition used before the ink composition is printed on a skin; a fixer composition used after the ink composition is printed on the skin; one or more of the ink composition, the ink fixer composition, and the fixer composition.

In addition, the present invention may provide a tattoo printing device including an ink composition for tattoo printing, and a tattoo printing kit may include the tattoo printing device.

In addition, the present invention provides a tattoo printing method including: applying an ink fixer agent composition to a skin; applying an ink composition to the skin on top of an area where the ink fixer composition has been applied; and applying a fixer composition to top of an area where the ink composition has been applied. However, the tattoo printing method may omit some of the steps or include additional steps.

The present invention is not limited to the above-described embodiments, and it is obvious that a combination of the embodiments or a combination of at least one of the embodiments and a known technology may be included as another embodiment.

Although the present invention has been described in detail through specific embodiments, this is intended to specifically explain the present invention, and the present invention is not limited thereto, and it will be apparent that modifications or improvements can be made by those skilled in the art within the technical spirit of the present invention.

All simple modifications or changes of the present invention fall within the scope of the present invention, and the specific protection scope of the present invention will be made clear by the appended claims.

## Claims

1. A tattoo printing method, wherein an ink fixer composition for tattoo printing including powder is applied before an ink composition for tattoo printing is applied or after an ink composition for tattoo printing is applied.

2. The tattoo printing method according to claim 1, wherein the ink fixer composition for tattoo printing includes a binder in an amount of 3% to 15% by weight based on the total weight of the ink fixer composition.

3. The tattoo printing method according to claim 1, wherein the powder includes spherical powder or plate-shaped powder.

4. The tattoo printing method according to claim 3, wherein the ink fixer composition for tattoo printing includes spherical powder in an amount of 54% to 89% by weight or plate-shaped powder in an amount of 5% to 35% by weight based on the total weight of the ink fixer composition.

5. The tattoo printing method according to claim 3, wherein the plate-shaped powder includes one or more of talc, mica, synthetic fluorophlogopite, boron nitride, sericite, illite, perlite, montmorillonite, boron nitride, bismuth oxychloride, barium sulfate, and alumina.

6. The tattoo printing method of claim 3, wherein the spherical powder has an average particle size of 3 to 10 µm.

7. The tattoo printing method according to claim 3, wherein the spherical powder includes one or more of silica, polymethyl methacrylate, methyl methacrylate crosspolymer, nylon-12, polymethylsilsesquioxane, vinyl dimethicone/methicone silsesquioxane crosspolymer, diphenyl dimethicone/vinyl diphenyl dimethicone/silsesquioxane crosspolymer, and hexamethylene diisocyanate (HDI)/trimethylol hexyllactone crosspolymer.

8. The tattoo printing method according to claim 3, wherein the spherical powder includes an absorbent spherical powder.

9. The tattoo printing method according to claim 8, wherein the absorbent spherical powder includes silica.

10. The tattoo printing method according to claim 8, wherein the absorbent spherical powder includes 16% or more of spherical powder having a sum of a water absorption amount and an oil absorption amount of 4 ml/g or more based on the total weight of the spherical powder.

11. The tattoo printing method according to claim 8, wherein the absorbent spherical powder has a sum of a water absorption amount and an oil absorption amount of 0.8 ml/g or more.

12. The tattoo printing method according to claim 8, wherein the absorbent spherical powder has a water absorption amount of 0.4 ml/g or more and an oil absorption amount of 0.45 ml/g or more.

13. An ink fixer composition for tattoo printing, comprising powder.

14. The ink fixer composition for tattoo printing according to claim 13, including a binder in an amount of 3% to 15% by weight based on the total weight of the ink fixer composition.

15. The ink fixer composition for tattoo printing according to claim 13, wherein the powder includes spherical powder or plate-shaped powder.

16. The ink fixer composition for tattoo printing according to claim 15, wherein the ink fixer composition for tattoo printing includes spherical powder in an amount of 54% to 89% by weight or plate-shaped powder in an amount of 5% to 35% by weight based on the total weight of the ink fixer composition.

17. The ink fixer composition for tattoo printing according to claim 15, wherein the plate-shaped powder includes one or more of talc, mica, synthetic fluorophlogopite, boron nitride, sericite, illite, perlite, montmorillonite, boron nitride, bismuth oxychloride, barium sulfate, and alumina.

18. The ink fixer composition for tattoo printing according to claim 15, wherein the spherical powder has an average particle size of 3 to 10 µm.

19. The ink fixer composition for tattoo printing according to claim 15, wherein the spherical powder includes one or more of silica, polymethyl methacrylate, methyl methacrylate crosspolymer, nylon-12, polymethylsilsesquioxane, vinyl dimethicone/methicone silsesquioxane crosspolymer, diphenyl dimethicone/vinyl diphenyl dimethicone/silsesquioxane crosspolymer, and HDI/trimethylol hexyllactone crosspolymer.

20. The ink fixer composition for tattoo printing according to claim 15, wherein the spherical powder includes an absorbent spherical powder.

21. The ink fixer composition for tattoo printing according to claim 20, wherein the absorbent spherical powder includes silica.

22. The ink fixer composition for tattoo printing according to claim 20, wherein the absorbent spherical powder includes 16% or more of spherical powder having a sum of a water absorption amount and an oil absorption amount of 4 ml/g or more based on the total weight of the spherical powder.

23. The ink fixer composition for tattoo printing according to claim 20, wherein the absorbent spherical powder has a sum of a water absorption amount and an oil absorption amount of 0.8 ml/g or more.

24. The ink fixer composition for tattoo printing according to claim 20, wherein the absorbent spherical powder has a water absorption amount of 0.4 ml/g or more and an oil absorption amount of 0.45 ml/g or more.

25. A tattoo printing kit comprising:
the ink fixer composition for tattoo printing according to any one of claims 13 to 24; and
a tattoo printing device including an ink composition for tattoo printing.

26. The tattoo printing kit according to claim 25, further comprising:
a top coat composition for tattoo printing or a fixer composition for tattoo printing.

27. A tattoo printing method comprising:
applying the ink fixer composition for tattoo printing according to any one of claims 13 to 24 to a skin; and
applying an ink composition for tattoo printing on top of an area on which the ink fixer composition for tattoo printing has been applied.

28. The tattoo printing method according to claim 27, comprising:
applying a top coat composition for tattoo printing or a fixer composition on top of an area on which the ink composition for tattoo printing has been applied.

29. An ink composition for tattoo printing, comprising:
one or more humectant selected from glycerin and polyethylene glycol (PEG)-8;
one or more surfactant selected from polyglyceryl-4 caprate and PEG-7 glyceryl cocoate; or
one or more antifoamer selected from glycereth-26 and a plant-derived oil.

30. The ink composition for tattoo printing according to claim 29, including the humectant in an amount of 10% to 40% by weight based on the total weight of the ink composition.

31. The ink composition for tattoo printing according to claim 29, including the surfactant in an amount of 0.001% to 10% by weight based on the total weight of the ink composition.

32. The ink composition for tattoo printing according to claim 29, including the antifoamer in an amount of 0.001% to 10% by weight based on the total weight of the ink composition.

33. The ink composition for tattoo printing according to claim 29, wherein the plant-derived oil includes one or more of hydrogenated ethylhexyl olivate and hydrogenated olive oil unsaponifiable.

34. The ink composition for tattoo printing according to claim 29, wherein the plant-derived oil is included in an amount of 0.001% to 10% by weight based on the total weight of the ink composition.

35. The ink composition for tattoo printing according to claim 29, wherein the ink composition is a water-based ink.

36. The ink composition for tattoo printing according to claim 29, further comprising a colorant including a dye or a pigment.

37. The ink composition for tattoo printing according to claim 36, wherein the colorant is included in an amount of 0.1% to 10% by weight based on the total weight of the ink composition.

38. A tattoo printing method comprising:
applying a primer composition for tattoo printing or an ink fixer composition for tattoo printing on a skin; and
applying the ink composition for tattoo printing according to any one of claims 29 to 37 on top of an area on which the primer composition for tattoo printing or the ink fixer composition for tattoo printing has been applied.

39. The tattoo printing method according to claim 38, further comprising:
applying a top coat composition for tattoo printing or a fixer composition for tattoo printing on top of an area on which the ink composition for tattoo printing has been applied.

40. A tattoo printing method, comprising:
applying the ink composition for tattoo printing according to any one of claims 29 to 37 on a skin; and
applying a top coat composition for tattoo printing or a fixer composition for tattoo printing on top of an area on which the ink composition for tattoo printing has been applied.

41. A tattoo printing device comprising: the ink composition for tattoo printing according to any one of claims 29 to 37.

42. A tattoo printing kit comprising:
the tattoo printing device according to claim 41; and
a primer composition for tattoo printing or an ink fixer composition for tattoo printing.

43. The tattoo printing kit according to claim 42, further comprising:
a top coat composition for tattoo printing or a fixer composition for tattoo printing.

44. A tattoo printing kit comprising:
the tattoo printing device according to claim 41; and
a top coat composition for tattoo printing or a fixer composition for tattoo printing.

45. A tattoo printing method in which a fixer composition for tattoo printing in any one formulation of a balm type, a jelly type, a solid type, and a semi-solid type is applied before an ink composition for tattoo printing is applied or after an ink composition for tattoo printing is applied.

46. The tattoo printing method according to claim 45, wherein the fixer composition for tattoo printing includes a hydrocarbon-based oil or wax.

47. The tattoo printing method according to claim 46, wherein the hydrocarbon oil or wax includes one or more of petrolatum, synthetic wax, ceresin, and microcrystalline wax.

48. The tattoo printing method according to claim 46, wherein the fixer composition for tattoo printing includes the hydrocarbon-based oil or wax in an amount of 65% to 100% by weight based on the total weight of the fixer composition.

49. A fixer composition for tattoo printing in any one formulation of a balm type, a jelly type, a solid type, and a semi-solid type.

50. The fixer composition for tattoo printing according to claim 49, including a hydrocarbon-based oil or wax.

51. The fixer composition for tattoo printing according to claim 50, wherein the hydrocarbon oil or wax include one or more of petrolatum, synthetic wax, ceresin, and microcrystalline wax.

52. The fixer composition for tattoo printing according to claim 50, including the hydrocarbon-based oil or wax in an amount of 65% to 100% by weight based on the total weight of the fixer composition.

53. A tattoo printing kit comprising:
the fixer composition for tattoo printing according to any one of claims 49 to 52; and
a tattoo printing device including an ink composition for tattoo printing.

54. A tattoo printing kit according to claim 53, further comprising: a primer composition for tattoo printing or an ink fixer composition for tattoo printing.

55. A tattoo printing method comprising:
applying an ink composition for tattoo printing to a skin; and
applying the fixer composition for tattoo printing of any one of claims 49 to 52 on top of an area on which the ink composition for tattoo printing has been applied.

56. A tattoo printing method according to claim 55, wherein the ink composition for tattoo printing is applied on top of an area on which a primer composition for tattoo printing or an ink fixer composition for tattoo printing has been applied.
